# EUROPEAN PATENT SPECIFICATION

(11) **EP 3 590 336 B1**
(45) Date of publication and mention of the grant of the patent: **26.02.2025**
(21) Application number: 19190267.5
(22) Date of filing: 27.11.2013
(51) Int. Cl.: A01N 33/04, A01N 33/06, A01N 33/12, A01P 1/00, A61L 2/16, C11D 7/26, C11D 7/32

(54) **USE OF A DISINFECTANT CLEANER COMPOSITION AGAINST ADENOVIRUS AND SIMIAN VIRUS 40**
VERWENDUNG EINER DESINFIZIERENDEN REINIGUNGSZUSAMMENSETZUNG GEGEN ADENOVIREN UND SIMIANVIREN 40
UTILISATION D'UNE COMPOSITION NETTOYANTE DESINFECTANTE CONTRE L'ADENOVIRUS ET LE VIRUS SIMIEN 40

(43) Date of publication of application: 08.01.2020
(62) Divisional of application: 13798316.9
(73) Proprietor: Ecolab USA Inc., St. Paul, MN 55102 (US)
(72) Inventor: LOHRMANN, Elke, 51429 Bergisch Gladbach (DE); KURZAWE, Christoph, 41469 Neuss (DE); MEYER, Bernhard, 40822 Mettmann (DE)
(74) Representative: Michalski Hüttermann & Partner Patentanwälte mbB

(56) References cited:
- EP-A1- 0 343 605
- DE-A1- 102006 006 765
- DE-A1- 19 526 481
- FR-A1- 2 383 671
- US-A- 4 797 420
- US-A- 4 983 635
- US-A1- 2012 225 948

## Description

### Field of the Invention

The present invention is directed to the non-therapeutic use of a disinfectant cleaner composition for inactivating and/or reducing viruses selected from Adenovirus and Simianvirus 40. In particular, the composition comprises an N-alkyl dimethyl benzyl ammonium chloride disinfectant that typically does not have tuberculocidal efficacy, but in association with bis(3-aminopropyl) alkylamine, surprisingly it is very effective in inactivating the above mentioned viruses.

### Background of the Invention

Microbes may often be present on many common objects and surfaces in everyday life. Microbes can include, for example, bacteria, fungi, spores, viruses, prions, microorganisms such as, e.g., Mycobacterium tuberculosis, listeria monocytogenas, escherichia coli, pseudomonas aeruginosa, salmonella typhimurium, salmonella enteritidis, Yersinia pestis, staphylococcus aureus, bacillus subtilis, enterobacter aerogenes, streptococcus faecalis, legionella pneumophila, vibrio parahaemolyticus, bacillus cereus, and other gram positive and gram negative bacteria. Several such microbes/ microorganisms, individually or in combination, can cause illness or other health problems, for example, when they come into contact with humans and/or animals, or when they are ingested along with food which has contacted them. These microbes present health hazards due to infection or contamination. When microorganisms are present on the surface of a substrate they can replicate rapidly to form colonies. The microbial colonies form a coating on the substrate surface which is known as a biofilm. Biofilms frequently consist of a number of different species of microorganisms which in turn can be snore difficult to eradicate and thus more hazardous to health than individual microorganisms. Some microorganisms also produce polysaccharide coatings, which makes them more difficult to destroy.

For example, hospitals and other medical facilities may have a particular need for sterile and/or uncontaminated surfaces, both in surgical and general medical areas as well as in convalescence facilities, where patient exposure may be significant and resistance to such microbes may be lowered.

Much time and effort can be spent, for example, on disinfecting and sterilizing medical instruments, testing devices. Often, such devices can be provided with disposable components or covers (e.g., disposable thermometer probes) to avoid cross-contamination between patients. Disposable needles are also commonly used. Such disposable materials involve increased costs and increased waste, as well as potential safety issues associated with their disposal.

The food-preparation and delivery industry is another area in which presence of microbes (e.g., Bacteria) can be problematic. Food preparation facilities, if contaminated with microbes, can lead to contamination of food which may cause health problems when ingested. For example, restaurants, food manufacturing plants, and even home kitchens can contain preparation surfaces, utensils, and equipment which may contaminate food that comes into contact with them. There may be, for example, a particular need for reducing a presence and spreading of microbes in meat packaging plants.

Public and private facilities such as, e.g., restrooms, may also contain surfaces which can harbor and spread microbes, leading to potential health problems. To address this issue, products such as antimicrobial soaps and air dryers for hands may be offered, as well as disposable paper towels. Nevertheless, microbes may still be harbored on such objects as faucet and toilet handles, door knobs, keys, dispenser levers.

In the transportation industry, including land, sea, air, and space vehicles, there may also be particular surfaces which can harbor and spread microbes, leading to potential health problems. For example, rental cars may benefit from disinfecting frequently touched surfaces. In particular, isolated environments such as, e.g., airplanes and submarines can also be safer if surfaces are disinfected.

Other common objects may benefit from antimicrobial treatments, which can inhibit or prevent spread of microorganisms between people and/or animals that come into contact with such objects. For example, musical instruments, such as harmonicas, flutes, clarinets, computer peripherals, communications equipment such as, e.g., telephones, pet accessories such as leashes and carriers, and/or other common household objects could benefit from antimicrobial surfaces.

U.S. Pat. No. 8,455,551 relates to a phenol-free broad spectrum disinfectant cleaner composition comprising an alkyl ammonium halogen, a solvent, an alkaline agent, a chelant, a nonionic surfactant coupler, and wetting and emulsifying surfactants that is effective in eradicating microorganisms such as Aspergillus niger and Aspergillus brasiiiensis. However, the phenol-free broad spectrum disinfectant cleaner composition is not effective in inactivating viruses such as Norovirus, Adenovirus and Polyomavirus. Further the described phenol-free broad spectrum disinfectant cleaner composition having no tuberculocidal efficacy.

EP 0 343 605 A1 refers to the use of N, N-Bis-(3-aminopropyl)-laurylamine as effective components in a liquid aldehyde-free composition active against tuberculosis.

DE 195 26 481 A1 refers to a powdered aldehyde and phenol free cleansing instrument disinfectants containing N, N-bis (3-aminopropyl) lauryl amine, a quaternary ammonium compound, a powdered complexing and a powdery, chemically neutral carrier substance.

FR 2 383 671 A1 refers to disinfectants effective against mycobacterium tuberculosis containing germicidal quaternary ammonium salt and an alkoxylated amine compound.

US 2012/225948 A1 refers to a broad spectrum disinfectant includes a quaternary ammonium halogen, an alkaline agent, a chelating agent, a nonionic surfactant coupler, at least one alkoxylated nonionic surfactant, and water or any aliphatic alcohol. The disinfectant composition is phenol-free, is effective in eradicating microorganisms such as various fungi, and is stable to gamma-irradiation.

US 4 797 420 A refers to a formulation effective against microorganisms including herpes simplex types 1 and 2 viruses, comprising an alkyl (58% C14, 28% C16, 14% C12) dimethyl benzyl ammonium chloride.

US 4 983 635 A refers to a quaternary ammonium compound with dual synergistic phenols is provided for disinfection and sanitization. This formula consists of dual chain quaternary ammonium (N-alkyldimethylethylbenzyl ammonium chloride N-alkyldimethylbenzyl ammonium chloride), ortho phenyl phenol, paratertiary amyl phenol, isopropyl alcohol, citric acid, and water.

DE 10 2006 006765 A1 refers to an alkaline disinfectant and cleaning agent comprising one or more selected quaternary ammonium salts, one or more selected alcohol alkoxylates and one or more alkylamines. By selection of the ingredients, an agent is provided which can be formulated as a concentrate and which, as a diluted aqueous working solution, has a cleaning performance in combination with a microbicidal activity. Quaternary ammonium chloride-containing products (also known as "quats") have been used in hard surface disinfection for many years. As a broad-spectrum disinfectant, they have acceptable efficacy against some organisms (e.g. Staphylococcus aureus), but often fall short in efficacy against mycobacteria and viruses. Thus, other harsher disinfectant or sporicidal products, such as Sodium chlorite (NaClO₂), ozone, H₂O₂ and peracetic acids are often used when mycobactericidal and virucidal efficacy is required.

Compositions and procedures have been developed to reduce or prevent a presence of microbes on certain surfaces. More importantly, such compositions may often not provide a rapid and high level of microbial efficacy, in particular a sufficient virucidal efficacy which could reduce the risk of microbial contaminants. Further, peroxide or chlorite containing antimicrobial compositions may have a material destructive effect on surfaces.

One can see that there is a continuing need for improved antimicrobial cleaners that are material non-destructive and having a rapid and high level of tuberculocidal and mycobactericidal efficacy and a rapid and high level of efficacy against Norovirus, Adenovirus and Polyomavirus.

### Summary of the Invention

The present invention is directed to the non-therapeutic use of a disinfectant cleaner composition for inactivating and/or reducing viruses on a hard and/or soft surface, wherein the viruses are selected from Adenovirus and Simianvirus 40 the composition comprising:
a) at least one quaternary ammonium chloride of N-alkyl dimethyl benzyl ammonium chloride, wherein said alkyl has from 8 to 18 carbon atoms
b) at least one bis(3-aminopropyl) alkylamine, wherein said alkyl has from 6 to 18 carbon atoms;
wherein the weight-% ratio of a) a quaternary ammonium chloride of N-alkyl dimethyl benzyl ammonium chloride, wherein said alkyl has from 8 to 18 carbon atoms to b) bis(3-aminopropyl) alkylamine, wherein said alkyl has from 6 to 18 carbon atoms, is in the range of > 0.1 : 1 to < 1 : 1.

It has been surprisingly found that using a disinfectant cleaner composition comprising a) a quaternary ammonium chloride of N-alkyl dimethyl benzyl ammonium chloride, wherein said alkyl has from 8 to 18 carbon atoms, to b) bis(3-aminopropyl) alkylamine, wherein said alkyl has from 6 to 18 carbon atoms, preferably bis(3-aminopropyl) dodecylamine, in a ratio of > 0.1 : 1 to < 1 : 1 is material non-destructive and having a rapid and high level of antiviral efficacy against Adenovirus, and Simianvirus 40.

According to one aspect of the invention the weight ratio of a) a quaternary ammonium chloride of N-alkyl dimethyl benzyl ammonium chloride, wherein said alkyl has from 8 to 18 carbon atoms, to b) bis(3-aminopropyl) alkylamine, wherein said alkyl has from 6 to 18 carbon atoms, preferably bis(3-aminopropyl) dodecylamine, can be in the range of ≥ 0.2 : 1 to ≤ 0.99 : 1, preferably of > 0.3 : 1 to ≤ 0.95 : 1, and further preferred of ≥ 0.4 : 1 to ≤ 0.95 : 1 and in addition preferred of > 0.5 : 1 to ≤ 0.95 : 1.

The disinfectant cleaner composition is used for inactivating and/or reducing viruses selected from Adenovirus, and Simianvirus 40, on hard and/or soft surfaces.

### Definition of terms

So that the invention maybe more readily understood, certain terms are first defined.

The disinfectant cleaner composition having tuberculocidal and mycobactericidal effectivity according to EN 14348 and/or in a surface test according to DGHM guideline 2001.

As used herein, "weight percent", "wt-%", "percent by weight", "% by weight", and variations thereof refer to a composition, component, substance or agent as the weight of that composition, component, substance or agent of the disinfectant cleaner composition divided by the total weight of the disinfectant cleaner composition or use composition and multiplied by 100. It is understood that the total weight percent amount of all components, substances or agents of the disinfectant cleaner composition as well as use composition are selected such that it does not exceed 100 wt.-%.

It is understood that, as used here, "percent", ,,%" are intended to be synonymous with "weight percent", "wt-%".

It should be noted that, as used in this specification and the appended claims, the singular forms "a", "an", and "the" include plural referents unless the content clearly dictates otherwise.

It should be noted that, as used in this specification and the appended claims, the term "N-dodecyl dimethyl benzyl ammonium chloride" stands for N-dodecyl dimethyl benzyl ammonium chloride or a mixture of quaternary ammonium chloride comprising N-alkyl dimethyl benzyl ammonium with C12 to C18 alkyl containing N-dodecyl dimethyl benzyl ammonium chloride as the main component of at least > 50% wt.-%, based on the total amount of N-alkyl dimethyl benzyl ammonium with C12 to C18 alkyl.

It should also be noted that the term "or" is generally employed in its sense including "and/or" unless the content clearly dictates otherwise.

### Disinfectant composition

According to one aspect the disinfectant cleaner composition can comprise in addition a phenoxy alkanol, wherein the alkanol has from 1 to 6 carbon atoms, or any combination thereof, and most preferred the phenoxy alkanol is phenoxy ethanol.

It has surprisingly been found that the efficacy against Adenovirus can be further increased by the addition of at least one phenoxy alkanol to the disinfectant composition.

The disinfectant cleaner composition may comprise:
a) at least one quaternary ammonium chloride of N-alkyl dimethyl benzyl ammonium chloride, wherein said alkyl has from 8 to 18 carbon atoms, further preferred dodecyl dimethyl benzyl ammonium chloride;
b) at least one bis(3-aminopropyl) alkylamine, wherein said alkyl has from 6 to 18 carbon atoms, preferably bis(3-aminopropyl) dodecylamine;
c) at least one phenoxy alkanol, preferably phenoxyethanol, wherein the alkanol has from 1 to 6 carbon atoms, or any combination thereof; and wherein
the weight ratio of a) a quaternary ammonium chloride of N-alkyl dimethyl benzyl ammonium chloride, wherein said alkyl has from 8 to 18 carbon atoms, further preferred dodecyl dimethyl benzyl ammonium chloride, to b) bis(3-aminopropyl) alkylamine, wherein said alkyl has from 6 to 18 carbon atoms, preferably bis(3-aminopropyl) dodecylamine, is in the range of > 0.1 : 1 to < 1 : 1.

The disinfectant cleaner composition may comprise in addition at least one alkali source, preferably selected from the group comprising alkali metal hydroxides, alkali metal salts and/or amines and mixtures thereof, preferably triethanol amine, sodium hydroxide, potassium hydroxide, sodium carbonate, and/or sodium bicarbonate and mixtures thereof and more preferred ethanolamine.

According to one aspect, the disinfectant cleaner composition may comprise:
a) at least one quaternary ammonium chloride of N-alkyl dimethyl benzyl ammonium chloride, wherein said alkyl has from 8 to 18 carbon atoms, further preferred dodecyl dimethyl benzyl ammonium chloride;
b) at least one bis(3-aminopropyl) alkylamine, wherein said alkyl has from 6 to 18 carbon atoms, preferably bis(3-aminopropyl) dodecylamine;
c) at least one phenoxy alkanol, preferably phenoxyethanol, wherein the alkanol has from 1 to 6 carbon atoms, or any combination thereof;
d) at least one alkali source; and wherein
the weight ratio of a) a quaternary ammonium chloride of N-alkyl dimethyl benzyl ammonium chloride, wherein said alkyl has from 8 to 18 carbon atoms, further preferred dodecyl dimethyl benzyl ammonium chloride, to b) bis(3-aminopropyl) alkylamine, wherein said alkyl has from 6 to 18 carbon atoms, preferably bis(3-aminopropyl) dodecylamine, is in the range of > 0.1 : 1 to < 1 : 1.

According to another aspect, the disinfectant cleaner composition may comprise in addition at least one nonionic surfactant. The surfactant may increase the penetration of the active components thus has a beneficial effect of the disinfectant efficacy.

According to one aspect, the disinfectant cleaner composition may comprise:
a) at least one quaternary ammonium chloride of N-alkyl dimethyl benzyl ammonium chloride, wherein said alkyl has from 8 to 18 carbon atoms, further preferred dodecyl dimethyl benzyl ammonium chloride;
b) at least one bis(3-aminopropyl) alkylamine, wherein said alkyl has from 6 to 18 carbon atoms, preferably bis(3-aminopropyl) dodecylamine;
c) at least one phenoxy alkanol, wherein the alkanol has from 1 to 6 carbon atoms, preferably phenoxyethanol, or any combination thereof;
d) optional at least one alkali source;
e) at least one nonionic surfactant; and wherein
the weight ratio of a) a quaternary ammonium chloride of N-alkyl dimethyl benzyl ammonium chloride, wherein said alkyl has from 8 to 18 carbon atoms, further preferred dodecyl dimethyl benzyl ammonium chloride, to b) bis(3-aminopropyl) alkylamine, wherein said alkyl has from 6 to 18 carbon atoms, preferably bis(3-aminopropyl) dodecylamine, is in the range of > 0.1 : 1 to < 1 : 1.

According to another aspect, the disinfectant cleaner composition may comprise in addition at least one corrosion inhibitor. The disinfection composition is a mild composition and possesses at the most a low corrosive potential with respect to metal surfaces, such as metal surgical instruments. In order to minimize the corrosive potential of the disinfection composition of the invention to a minimum at least one corrosion inhibitor can be added.

According to one aspect, the disinfectant cleaner composition may comprise:
a) at least one quaternary ammonium chloride of N-alkyl dimethyl benzyl ammonium chloride, wherein said alkyl has from 8 to 18 carbon atoms, further preferred dodecyl dimethyl benzyl ammonium chloride;
b) at least one bis(3-aminopropyl) alkylamine, wherein said alkyl has from 6 to 18 carbon atoms, preferably bis(3-aminopropyl) dodecylamine;
c) at least one phenoxy alkanol, wherein the alkanol has from 1 to 6 carbon atoms, preferably phenoxyethanol, or any combination thereof;
d) optional at least one alkali source;
e) optional at least one nonionic surfactant;
f) at least one corrosion inhibitor; and wherein
the weight ratio of a) a quaternary ammonium chloride of N-alkyl dimethyl benzyl ammonium chloride, wherein said alkyl has from 8 to 18 carbon atoms, further preferred dodecyl dimethyl benzyl ammonium chloride, to b) bis(3-aminopropyl) alkylamine, wherein said alkyl has from 6 to 18 carbon atoms, preferably bis(3-aminopropyl) dodecylamine, is in the range of > 0.1 : 1 to < 1 : 1.

According to another aspect, the disinfectant cleaner composition may comprise in addition at least one sequestering agent. The sequestering agents may remove contaminants, such as iron, manganese, calcium and minimizing color, scale, deposits and/or corrosion.

According to one aspect, the disinfectant cleaner composition may comprise:
a) at least one quaternary ammonium chloride of N-alkyl dimethyl benzyl ammonium chloride, wherein said alkyl has from 8 to 18 carbon atoms, further preferred dodecyl dimethyl benzyl ammonium chloride;
b) at least one bis(3-aminopropyl) alkylamine, wherein said alkyl has from 6 to 18 carbon atoms, preferably bis(3-aminopropyl) dodecylamine;
c) at least one phenoxy alkanol, wherein the alkanol has from 1 to 6 carbon atoms, preferably phenoxyethanol, or any combination thereof;
d) optional at least one alkali source;
e) optional at least one nonionic surfactant;
f) optional at least one corrosion inhibitor;
g) at least one sequestering agent and wherein
the weight ratio of a) a quaternary ammonium chloride of N-alkyl dimethyl benzyl ammonium chloride, wherein said alkyl has from 8 to 18 carbon atoms, further preferred dodecyl dimethyl benzyl ammonium chloride, to b) bis(3-aminopropyl) alkylamine, wherein said alkyl has from 6 to 18 carbon atoms, preferably bis(3-aminopropyl) dodecylamine, is in the range of > 0.1 : 1 to < 1 : 1.

According to another aspect, the disinfectant cleaner composition may be in form of a liquid composition. Preferably, the disinfectant cleaner composition may be in form of a concentrated liquid composition. However, the disinfectant cleaner composition may be in form of a diluted liquid composition, that can be directly applied on the surface to be disinfected, also referred to as "ready-to-use" solution or "ready-to-use" composition.

According to one aspect, the disinfectant cleaner composition may comprise:
a) at least one quaternary ammonium chloride of N-alkyl dimethyl benzyl ammonium chloride, wherein said alkyl has from 8 to 18 carbon atoms, further preferred dodecyl dimethyl benzyl ammonium chloride;
b) at least one bis(3-aminopropyl) alkylamine, wherein said alkyl has from 6 to 18 carbon atoms, preferably bis(3-aminopropyl) dodecylamine;
c) at least one phenoxy alkanol, wherein the alkanol has from 1 to 6 carbon atoms, preferably phenoxyethanol, or any combination thereof;
d) optional at least one alkali source;
e) optional at least one nonionic surfactant;
f) optional at least one corrosion inhibitor;
g) optional at least one sequestering agent:
h) at least one solvent; and wherein
the weight ratio of a a) a quaternary ammonium chloride of N-alkyl dimethyl benzyl ammonium chloride, wherein said alkyl has from 8 to 18 carbon atoms, further preferred dodecyl dimethyl benzyl ammonium chloride, to b) bis(3-aminopropyl) alkylamine, wherein said alkyl has from 6 to 18 carbon atoms, preferably bis(3-aminopropyl) dodecylamine, is in the range of > 0.1 : 1 to < 1 : 1.

### Components used in the disinfectant cleaner composition used in the Invention

The preferred compositions comprise ingredients, which are cooperative in their combined effects and effective in inactivating and/or reducing infectious agents, comprising bacteria, virus, and/or yeasts. Thus, the disinfectant cleaner composition of has for example tuberculocidal or mycobactericidal effectivity.

The disinfectant cleaner composition can be free of nonionic tensides, cationic tensides except N-alkyl dimethyl benzyl ammonium chloride and/or amphoteric tensides.

The disinfectant cleaner composition can be free of a nonionic surfactant coupler and/or alkoxylated nonionic surfactant, or both.

It should be understood that the disinfectant cleaner composition can be free of a hydrotrope component.

It should be understood that the disinfectant cleaner composition can be free of a phosphate.

It should be understood that the disinfectant cleaner composition can be free of at least one additive, preferably all additives, selected from the group fragrances, dyes, antistatic agents, UV absorbers, reducing agents and/or buffering compounds.

### N-alkyl dimethyl benzyl ammonium chloride

The disinfectant cleaner composition comprises at least one N-alkyl dimethyl benzyl ammonium chloride. The N-alkyl dimethyl benzyl ammonium chloride, wherein said alkyl has from 8 to 18 carbon atoms, also known as benzalkonium chloride and ADBAC, is a cationic surface-acting agent belonging to the quaternary ammonium group. It has three main categories of use: as a biocide, a cationic surfactant, and phase transfer agent in the chemical industry.

The least one quaternary ammonium chloride can be:
at least one N-alkyl dimethyl benzyl ammonium chloride, wherein said alkyl has from 8 to 18 carbon atoms, can be selected from the group of at least one alkyl(C14) dimethyl benzyl ammonium chloride, alkyl(C16) dimethyl benzyl ammonium chloride, alkyl(C18) dimethyl benzyl ammonium chloride, a mixture of N-alkyl(C8-C18) dimethyl benzyl ammonium chloride, or a mixture of N-alkyl(C10-C18) dimethyl benzyl ammonium chloride, or any combination thereof; and more preferred a N-alkyl(C10-C18) dimethyl benzyl ammonium chloride mixture, further further preferred dodecyl dimethyl benzyl ammonium chloride and most preferred a N-alkyl(C10-C18) dimethyl benzyl ammonium chloride mixture, wherein the weight amount, based on the N-alkyl(C10-C18)dimethyl benzyl ammonium chloride mixture, of the N-dodecyl dimethyl benzyl ammonium chloride (C12) is > than N-quatrodecyl dimethyl benzyl ammonium chloride (C14) is > than N-hexadecyl dimethyl benzyl ammonium chloride (C16) is > than N-decyl dimethyl benzyl ammonium chloride (C10) > than N-octadecyl dimethyl benzyl ammonium chloride (C18).

A disinfectant cleaner composition used in the invention, preferably in form of a concentrate, may comprise ≥ 4 wt.-% to ≤ 11 wt.-%, preferably ≥ 5 wt.-% to ≤ 10 wt.-%, more preferred ≥ 6 wt.-% to ≤ 9 wt.-%, and most preferred of > 7 wt.-% to ≤ 8 wt.-% of:
- at least one C8 to C18 N-alkyl dimethyl benzyl ammonium chloride, preferably a mixture of a N-alkyl(C10-C18)dimethyl benzyl ammonium chloride, further preferred N-alkyl(C10-C18)dimethyl benzyl ammonium chloride, further further preferred dodecyl dimethyl benzyl ammonium chloride and/or more preferred a N-alkyl(C10-C18)dimethyl benzyl ammonium chloride mixture; based on the total weight amount of the disinfectant cleaner composition of the invention, preferably in form of a concentrate.

A diluted disinfectant cleaner composition used in the invention may comprise ≥ 0.005 wt.-% to ≤ 0.6 wt.-%, preferably ≥ 0.01 wt.-% to ≤ 0.5 wt.-%, more preferred ≥ 0.015 wt.-% to ≤ 0.4 wt.-%, and most preferred of > 0.015 wt.-% to ≤ 0.3 wt.-% of:
- at least one C8 to C18 N-alkyl dimethyl benzyl ammonium chloride, preferably a mixture of a N-alkyl(C10-C18)dimethyl benzyl ammonium chloride, further preferred N-alkyl(C10-C18)dimethyl benzyl ammonium chloride, further further preferred dodecyl dimethyl benzyl ammonium chloride and/or more preferred a N-alkyl(C10-C18)dimethyl benzyl ammonium chloride mixture; based on the total weight amount of the diluted disinfectant cleaner composition used in the invention.

### bis(3-aminopropyl) alkylamine

The disinfectant cleaner composition used in the invention comprises at least one bis(3-aminopropyl) alkylamine. The bis(3-aminopropyl) alkylamine, wherein said alkyl has from 6 to 18 carbon atoms, is known to be effective against bacteria, yeasts and moulds in low concentrations. The bis(3-aminopropyl) alkylamine, wherein said alkyl has from 6 to 18 carbon atoms, is not known to have antiviral efficacy against Norovirus, Adenovirus and/or Polyomavirus.

The bis(3-aminopropyl) alkylamine, wherein said alkyl has from 6 to 18 carbon atoms, may be selected from the group comprising a bis(3-aminopropyl) C6-C18-alkylamine, a bis(3-aminopropyl) octylamine, a bis(3-aminopropyl) decylamine, a bis(3-aminopropyl) dodecylamine, a bis(3-aminopropyl) quatrodecylamine, a bis(3-aminopropyl) hexadecylamine, a bis(3-aminopropyl) octadecylamine, or any combination thereof, and most preferred is a bis(3-aminopropyl) dodecylamine.

A disinfectant cleaner composition used in the invention, preferably in form of a concentrate, may comprise ≥ 4 wt.-% to ≤ 12 wt.-%, preferably ≥ 5 wt.-% to ≤ 11 wt.-%, more preferred ≥6 wt.-% to ≤ 10 wt.-%, and most preferred of ≥ 7 wt.-% to ≤ 9 wt.-% of at least one bis(3-aminopropyl) C8-C18 alkylamine, or any combination thereof, and most preferred bis(3-aminopropyl) dodecylamine; based on the total weight amount of the disinfectant cleaner composition used in the invention, preferably in form of a concentrate.

A diluted disinfectant cleaner composition of the invention may comprise ≥ 0.01 wt.-% to ≤ 0.6 wt.-%, preferably ≥ 0.015 wt.-% to ≤ 0.5 wt.-%, more preferred ≥ 0.02 wt.-% to ≤ 0.45 wt.-%, and most preferred of > 0.02 wt.-% to ≤ 0.4 wt.-% of at least one bis(3-aminopropyl) C8-C18 alkylamine, or any combination thereof, and most preferred bis(3-aminopropyl) dodecylamine; based on the total weight amount of the diluted disinfectant cleaner composition used in the invention.

### Phenoxy alkanol

The disinfectant cleaner composition used in the invention may comprise at least one phenoxy alkanol. The phenoxy alkanol, wherein the alkanol has from 1 to 6 carbon atoms, is known to be used in many applications such as cosmetics, vaccines and pharmaceuticals as a preservative. Phenoxy alkanol is not known to have antiviral efficacy against Norovirus, Adenovirus and/or Polyomavirus.

The phenoxy alkanol can be selected from the group comprising at least one phenoxy alkanol, wherein the alkanol has from 1 to 6 carbon atoms, preferably 2 to 4 or 3 carbon atoms, or any combination thereof, and most preferred the phenoxy alkanol is a phenoxy ethanol.

A disinfectant cleaner composition used in the invention, preferably in form of a concentrate, may comprise ≥ 0 wt.-% to ≤ 20 wt.-%, preferably ≥ 5 wt.-% to ≤ 15 wt.-%, more preferred ≥ 8 wt.-% to ≤ 12 wt.-%, and most preferred of > 9 wt.-% to ≤ 11 wt.-% of at least one phenoxy alkanol, wherein the alkanol has from 1 to 6 carbon atoms or any combination thereof, and most preferred phenoxy ethanol; based on the total weight amount of the disinfectant cleaner composition used in the invention, preferably in form of a concentrate.

A diluted disinfectant cleaner composition used in the invention may comprise ≥ 0 wt.-% to ≤ 1 wt.-%, preferably ≥ 0.01 wt.-% to ≤ 0.8 wt.-%, more preferred ≥ 0.02 wt.-% to ≤ 0.6 wt.-%, and most preferred of > 0.02 wt.-% to ≤ 0.5 wt.-% of at least one phenoxy alkanol, wherein the alkanol has from 1 to 6 carbon atoms or any combination thereof, and most preferred phenoxy ethanol; based on the total weight amount of the diluted disinfectant cleaner composition used in the invention.

### Surfactant

The disinfectant cleaner composition may include nonionic surfactants, cationic surfactants, amphoteric surfactants and mixtures thereof. The surfactant component can be used to reduce surface tension.

As used herein, "surfactant" refers to any agent that lowers the surface tension of a liquid, for example water. Exemplary surfactants which may be suitable for use with the present invention are described below. In one embodiment, surfactants may be selected from the group consisting of nonionic, cationic, amphoteric, zwitterionic, and combinations thereof.

Most preferred, the disinfectant cleaner composition is free of an anionic surfactant.

### Nonionic surfactant

Exemplary nonionic surfactants that can be used in the disinfectant cleaner composition of the invention may be alkoxylated, preferably ethoxylated or ethoxylated and propoxylated, fatty acid alkyl esters preferably containing 1 to 4 carbon atoms in the alkyl chain, more particularly the fatty acid methyl esters.

Further surfactants include ethoxylated long chain fatty acid amides where the fatty acid has 8-20 carbon atoms and the amide group is ethoxylated with 1-20 ethylene oxide units.

A further class of nonionic surfactants, which can be used according to the invention, is that of the alkyl polyglycosides (APG). Suitable alkyl polyglycosides satisfy the general Formula RO(G)z where R is a linear or branched, particularly 2-methyl-branched, saturated or unsaturated aliphatic radical containing 4 to 22 and preferably 6 to 18 carbon atoms and G stands for a glycose unit containing 5 or 6 carbon atoms, preferably glucose. The degree of oligomerization z is a number between 1.0 and 4.0 and preferably between 1.1 and 1.4.

Additionally, non-ionic surfactants derived from the condensation of ethylene oxide with the product resulting from the reaction of propylene oxide and ethylene diamine are also useful. For example, there are compounds containing from 40% to 80% of polyoxyethylene by weight and having a molecular weight from 5,000 to 11,000 resulting from the reaction of ethylene oxide groups with a hydrophobic base constituted of the reaction product from ethylene diamine and excess propylene oxide wherein the base has a molecular weight on order of 2,500-3, 000.

Suitable nonionic surfactants include the polyoxyethylene-polyoxypropylene condensates, which are sold by BASF under the trade name'Pluronic', polyoxyethylene condensates of aliphatic alcohols/ethylene oxide condensates having from 1 to 30 moles of ethylene oxide per mole of coconut alcohol; ethoxylated long chain alcohols sold by Shell Chemical Co. under the trade name 'Neodol', polyoxyethylene condensates of sorbitan fatty acids, alkanolamides, such as the monoalkoanolamides, dialkanolamides and the ethoxylated alkanolamides, for example coconut monoethanolamide, lauric isopropanolamide and lauric diethanolamide; and amine oxides for example dodecyldimethylamine oxide.

Nonionic surfactants that can be used in the composition used in the invention include polyalkylene oxide surfactants (also known as polyoxyalkylene surfactants or polyalkylene glycol surfactants). Suitable polyalkylene oxide surfactants include polyoxypropylene surfactants and polyoxyethylene glycol surfactants. Suitable surfactants of this type are synthetic organic polyoxypropylene (PO)-polyoxyethylene (EO) block copolymers. These surfactants include a di-block polymer comprising an EO block and a PO block, a center block of polyoxypropylene units (PO), and having blocks of polyoxyethylene grafted onto the polyoxypropylene unit or a center block of EO with attached PO blocks. Further, this surfactant can have further blocks of either polyoxyethylene or polyoxypropylene in the molecules. A suitable average molecular weight range of useful surfactants can be 1,000 to 40,000 and the weight percent content of ethylene oxide can be 10-80 wt %.

A suitable polyethylene glycol for use in the composition used in the invention can have an average mol weight (MW) in the range of ≥ 4000 to ≤ 12000, preferably ≥ 6000 to ≤ 10000 and more preferred of ≥ 7000 to ≤ 8000. Polyethylene glycol that can be used are marketed for example by BASF under the tradename PLURIOL^{®}.

The disinfectant cleaner composition may comprises at least one polyethylene glycol, preferably a polyethylene glycol with an average mol weight in the range of 4.000 to 12.000, and more preferred a polyethylene glycol having an average mol weight of 8,000.

Further exemplary non-ionic surfactants include alkylphenol alkoxylates, and amine oxides such as alkyl dimethylamine oxide or bis(2- hydroxyethyl) alkylamine oxide.

Most preferred, the disinfectant cleaner composition used in the invention may comprises at least one nonionic surfactant, preferably at least one C4 to C18 alkyl polyglycoside or any combination thereof, preferably at least one C8 to C16 alkyl polyglycoside and more preferred a mixture of C8 to C16 alkyl polyglycosides.

The disinfectant cleaner composition used in the invention, preferably in form of a concentrate, may comprises ≥ 0 wt.-% to ≤ 10 wt.-%, preferably ≥ 1 wt.-% to ≤ 8 wt.-%, more preferred ≥ 2 wt.-% to ≤ 6 wt.-%, and most preferred of ≥ 3 wt.-% to ≤ 5 wt.-% of at least one C4 to C18 alkyl polyglycoside or any combination thereof, preferably at least one C6 to C18 alkyl polyglycoside and more preferred a mixture of C8 to C16 alkyl polyglycosides; based on the total weight amount of the disinfectant cleaner composition used in the invention, preferably in form of a concentrate.

A diluted disinfectant cleaner composition used in the invention may comprise ≥ 0 wt.-% to ≤ 0.5 wt.-%, preferably ≥ 0.0025 wt.-% to ≤ 0.5 wt.-%, more preferred ≥ 0.005 wt.-% to ≤ 0.25 wt.-%, and most preferred of ≥ 0.01 wt.-% to ≤ 0.2 wt.-% of at least one C4 to C18 alkyl polyglycoside or any combination thereof, preferably at least one C6 to C18 alkyl polyglycoside and more preferred a mixture of C8 to C16 alkyl polyglycosides; based on the total weight amount of the diluted disinfectant cleaner composition used in the invention.

### Cationic surfactans

The disinfectant cleaner composition can contain a cationic surfactant component. The cationic surfactant can be used to provide sanitizing properties. Cationic surfactants that can be used in the disinfectant cleaner composition include: amines such as primary, secondary and tertiary monoamines with C1-8 alkyl or alkenyl chains, ethoxylated alkylamines, alkoxylates of ethylenediamine, imidazoles such as a 1-(2-hydroxyethyl)-2-imidazoline, a 2-alkyl-1-(2-hydroxyethyl)-2-imidazoline; and poly sulfonate ammonium salts, as for example, alkylpoly sulfonate ammonium chloride surfactants such as n-alkyl(C12-C18)dimethylbenzyl ammonium chloride, n-tetradecyldimethylbenzylammonium chloride monohydrate, and a naphthylene-substituted poly sulfonate ammonium chloride such as dimethyl-1-naphthylmethylammonium chloride. Suitable cationic surfactants include quaternary ammonium compounds having the formula of RR'R"R‴N⁺X⁻, where R, R', R" and R‴ are each a C₁-C₂₄ alkyl, aryl or arylalkyl group that can optionally contain one or more P, O, S or N heteroatoms, and X is F, Cl, Br, I or an alkyl sulfate.

Additional preferred cationic surfactants include ethoxylated and/or propoxylated alkyl amines, diamines, or triamines.

Each of R, R', R" and R‴ can independently include, individually or in combination, substituents including 6 to 24 carbon atoms, preferably 14 to 24 carbon atoms, and more preferably, 16 to 24 carbon atoms.

Each of R, R', R" and R‴ can independently be linear, cyclic, branched, saturated, or unsaturated, and can include heteroatoms such as oxygen, phosphorous, sulfur, or nitrogen. Any two of R, R', R" and R‴ can form a cyclic group. Any one of three of R, R', R" and R‴ can independently be hydrogen. X is preferably a counter ion and preferably a non-fluoride counter ion. Exemplary counter ions include chloride, bromide, methosulfate, ethosulfate, sulfate, and phosphate.

In an embodiment, the quaternary ammonium compound includes alkyl ethoxylated and/or propoxylated quaternary ammonium salts (or amines).

Preferably, the alkyl group contains between 6 and 22 carbon atoms and can be saturated and/or unsaturated. The degree of ethoxylation is preferably between 2 and 20, and/or the degree of propoxylation is preferably between 0 and 30.

In an embodiment, the quaternary ammonium compound includes an alkyl group with 6 to 22 carbon atoms and a degree of ethoxylation between 2 and 20. A preferred cationic surfactant is commercially available under the name Berol 563 from Akzo-Nobel.

The cationic surfactants can be provided in a disinfectant cleaner composition used in the invention, preferably in form of a concentrate, in ≥ 0 wt.-% to ≤ 10 wt.-%, preferably ≥ 1 wt.-% to ≤ 8 wt.-%, more preferred ≥ 2 wt.-% to ≤ 6 wt.-%, and most preferred of > 3 wt.-% to ≤ 5 wt.-%; based on the total weight amount of the disinfectant cleaner composition used in the invention, preferably in form of a concentrate.

A diluted disinfectant cleaner composition used in the invention may comprise ≥ 0 wt.-% to ≤ 0.5 wt.-%, preferably ≥ 0.0025 wt.-% to ≤ 0.5 wt.-%, more preferred ≥ 0.005 wt.-% to ≤ 0.25 wt.-%, and most preferred of ≥ 0.01 wt.-% to ≤ 0.2 wt.-% of at least one cationic surfactant; based on the total weight amount of the diluted disinfectant cleaner composition used in the invention.

It should be understood that the disinfectant cleaner composition used in the invention can be preferably free of a cationic surfactant except for the active components a) of at least one quaternary ammonium chloride of N-alkyl dimethyl benzyl ammonium chloride, wherein said alkyl has from 8 to 18 carbon atoms, further preferred dodecyl dimethyl benzyl ammonium chloride, and b) at least one bis(3-aminopropyl) alkylamine, wherein said alkyl has from 6 to 18 carbon atoms, preferably bis(3-aminopropyl)dodecylamine.

### Amphoteric Surfactants

Amphoteric surfactants can also be used to provide desired detersive properties. Suitable amphoteric surfactants that can be used include: betaines, imidazolines, and propionates. Suitable amphoteric surfactants include: sultaines, amphopropionates, amphodipropionates, aminopropionates, aminodipropionates, amphoacetates, amphodiacetates, and amphohydroxypropylsulfonates. When the disinfectant cleaner composition includes an amphoteric surfactant, the amphoteric surfactant can be included in an amount of ≥ 0 wt.-% to ≤ 10 wt.-%, preferably ≥ 1 wt.-% to ≤ 8 wt.-%, more preferred ≥ 2 wt.-% to ≤ 6 wt.-%, and most preferred of > 3 wt.-% to ≤ 5 wt.-% of at least one amphoteric surfactant; based on the total weight amount of the disinfectant cleaner composition used in the invention, preferably in form of a concentrate.

A diluted disinfectant cleaner composition used in the invention may comprise ≥ 0 wt.-% to ≤ 0.5 wt.-%, preferably ≥ 0.0025 wt.-% to ≤ 0.5 wt.-%, more preferred ≥ 0.005 wt.-% to ≤ 0.25 wt.-%, and most preferred of ≥ 0.01 wt.-% to ≤ 0.2 wt.-% of at least one amphoteric surfactant; based on the total weight amount of the diluted disinfectant cleaner composition used in the invention.

It should be understood that the disinfectant cleaner composition used in the invention can be preferably free of an amphoteric surfactant.

### Alkaline Source

The source of alkalinity can be any source of alkalinity that is compatible with the other components of the disinfectant cleaner composition and that will provide the solution with the desired pH.

Exemplary sources of alkalinity include alkali metal hydroxides, alkali metal salts, amines, and mixtures thereof.

Exemplary alkali metal hydroxides include sodium hydroxide, potassium hydroxide, and lithium hydroxide.

Exemplary alkali metal salts include sodium carbonate, trisodium phosphate, potassium carbonate, and mixtures thereof.

Exemplary amines include alkanolamine selected from the group comprising triethanolamine, monoethanolamine, diethanolamine, and mixtures thereof.

The source of alkalinity, preferably an alkali metal hydroxide, may be added to the disinfectant cleaner composition in a variety of forms, including for example in the form of solid beads, dissolved in an aqueous solution or a combination thereof. Alkali metal hydroxides are commercially available as pellets or beads having a mix of particle sizes ranging from 12-100 U. S. mesh, or as an aqueous solution, as for example, as 45 wt. %, 50 wt. % and 73 wt. % solution.

Preferably the alkalinity source is selected from the group comprising alkali metal hydroxides, alkali metal salts, phosphates and/or amines and mixtures thereof, preferably triethanol amine, sodium hydroxide, potassium hydroxide, sodium carbonate, and/or sodium bicarbonate and mixtures thereof and more preferred ethanolamine.

According to the invention, the disinfectant composition, preferably in form of a concentrate, may comprises ≥ 0 wt.-% to ≤ 10 wt.-%, preferably ≥ 0.1 wt.-% to ≤ 8 wt.-%, more preferred ≥ 0.5 wt.-% to ≤ 5 wt.-%, and most preferred of > 1 wt.-% to ≤ 3 wt.-% of at least one alkali source, and more preferred ethanolamine; based on the total weight amount of the disinfectant cleaner composition used in the invention, preferably in form of a concentrate.

A diluted disinfectant cleaner composition used in the invention may comprise ≥ 0 wt.-% to ≤ 0.5 wt.-%, preferably ≥ 0.00025 wt.-% to ≤ 0.4 wt.-%, more preferred ≥ 0.001 wt.-% to ≤ 0.2 wt.-%, and most preferred of ≥ 0.004 wt.-% to ≤ 0.1 wt.-% of at least one alkali source, and more preferred ethanolamine; based on the total weight amount of the diluted disinfectant cleaner composition used in the invention.

It should be understood that the disinfectant cleaner composition used in the invention can be preferably free of an alkali source.

It should be understood that the disinfectant cleaner composition used in the invention can be preferably free of phosphates.

### Corrosion inhibitor

At least one corrosion inhibitor maybe used to prevent the corrosion of an article to be cleaned.

Preferably a silicate(s) corrosion inhibitor and more preferred a disilicate corrosion inhibitor can be used in the disinfectant cleaner composition used in the present invention. The silicate(s) and/or disilicate corrosion inhibitor can be an alkali silicate and/or alkali disilicate.

Other inhibitors that can be used can be selected from the group comprising calcium acetate, calcium chloride, calcium gluconate, calcium phosphate, calcium borate, calcium carbonate, calcium citrate, calcium lactate, calcium sulfate, calcium tartrate, benzotriazole, 1,2,3-benzotriazole and mixtures thereof.

More preferred, the corrosion inhibitor is a heterocyclic compound, a triazole derivate, such as a benzotriazole or 1,2,3-benzotriazole and mixtures thereof.

Exemplary silicates include sodium metasilicates, sesquisilicates, orthosilicates, potassium silicates, and mixtures thereof. However, most preferred can be sodium silicate.

The silicates may comprise at least one crystalline layer-forming silicate of the general formula NaMSixO2x+1.yH2O, wherein M represents sodium or hydrogen, x is a number from 1.9 to 22, preferably 1.9 to 4 and y stands for a number from 0 to 33.

The crystalline layer-forming silicates of the formula NaMSixO22x+1.yH2O are marketed for example by Clariant GmbH (Germany) under the trade names Na-SKS, eg. Na-SKS-1 (Na2Si22O45.xH2O, Kenyait), Na-SKS-2 (Na2Si14O29.xH2O, Magadiit), Na-SKS-3 (Na2Si8O17.xH2O) or Na-SKS-4 (Na2Si4O9.xH2O, Makatit).

Crystalline, layered silicates of the above formula, in which x stands for 2, are particularly suitable for the purposes of the present invention.

Na-SKS-5 (alpha -Na2Si2O5), Na-SKS-7 (beta -Na2Si2O5, Natrosilit), Na-SKS-9 (NaHSi2O5.H2O), Na-SKS-10 (NaHSi2O5.3H2O, Kanemit), Na-SKS-11 (t-Na2Si2O5) and Na-SKS-13 (NaHSi2O5) are most notably suitable, particularly Na-SKS-6 (delta -Na2Si2O5).

In the context of the present application, silicates can comprise a content by weight of crystalline layered silicates of formula NaMSixO2x+1.yH2O of 0.1 to 20 wt. %, preferably 0.2 to 15 wt. % and particularly 0.4 to 10 wt. %, each based on the total weight of the corrosion inhibitor agent.

Particularly preferred are especially those that have a total silicate content > 0 and below 7 wt.- %, advantageously below 6 wt.- %, preferably below 5 wt.- %, particularly preferably below 4 wt.- %, quite particularly preferably below 3 wt. -% and especially below 2.5 wt.- %, wherein this silicate, based on the total weight of the comprised silicate, is advantageously at least 70 wt.- %, preferably at least 80 wt.- % and especially at least 90 wt.-% of a silicate of the general formula NaMSixO2x+1.yH2O.

However, other corrosion inhibitors can be suitable added to the disinfectant composition used in this invention include magnesium and/or zinc ions and Ca (NO₂)₂. Preferably, the metal ions are provided in water-soluble form.

Examples of useful water-soluble forms of magnesium and zinc ions are the water-soluble salts thereof including the chlorides, nitrates and sulfates of the respective metals. If any of the alkalinity providing agents are the alkali metal carbonates, bicarbonates or mixtures of such agents, magnesium oxide can be used to provide the Mg ion. The magnesium oxide is water soluble and is a preferred source of Mg ions.

In order to maintain the dispersibility of the magnesium and/or zinc corrosion inhibitors in aqueous solution, and in the presence of agents which would otherwise cause precipitation of the zinc or magnesium ions, e. g. , carbonates, it might be advantageous to include a carboxylated polymer to the solution.

The useful carboxylated polymer corrosion inhibitors may be generically categorized as water-soluble carboxylic acid polymers such as polyacrylic and polymethacrylic acids or vinyl addition polymers, in addition to the acid-substituted polymers used in the present invention.

Of the vinyl addition polymer corrosion inhibitors contemplated, maleic anhydride copolymers as with vinyl acetate, styrene, ethylene, isobutylene, acrylic acid and vinyl ethers are examples.

The polymers tend to be water-soluble or at least colloidally dispersible in water. The molecular weight of these polymers may vary over a broad range although it is preferred to use polymers having average molecular weights ranging between 1,000 up to 1,000, 000. These polymers have a molecular weight of 100,000 or less and between 1,000 and 10,000.

The polymers or copolymers (either the acid-substituted polymers or other added polymers) may be prepared by either addition or hydrolytic techniques. Thus, maleic anhydride copolymers are prepared by the addition polymerization of maleic anhydride and another comonomer such as styrene.

The low molecular weight acrylic acid polymer corrosion inhibitors may be prepared by addition polymerization of acrylic acid or its salts either with itself or other vinyl comonomers.

Alternatively, such polymers may be prepared by the alkaline hydrolysis of low molecular weight acrylonitrile homopolymers or copolymers.

According to a more preferred aspect, the disinfectant cleaner composition may comprises of at least one corrosion inhibitor selected from the group comprising silicate, sodium silicate, sodium disilicate, calcium acetate, calcium chloride, calcium gluconate, calcium phosphate, calcium borate, calcium carbonate, calcium citrate, calcium lactate, calcium sulfate, calcium tartrate, benzotriazole, 1,2,3-benzotriazole, or any combination thereof, more preferred at least one benzotriazole, and most preferred at least one 1,2,3-benzotriazole.

The disinfectant composition, preferably in form of a concentrate, may comprises ≥ 0 wt.-% to ≤ 1 wt.-%, preferably ≥ 0.05 wt.-% to ≤ 0.8 wt.-%, more preferred ≥ 0.1 wt.-% to ≤ 0.6 wt.-%, and most preferred of ≥ 0.15 wt.-% to ≤ 0.4 wt.-% of at least one corrosion inhibitor, preferably benzotriazole, and most preferred at least one 1,2,3-benzotriazole; based on the total weight amount of the disinfectant cleaner composition used in the invention, preferably in form of a concentrate.

A diluted disinfectant cleaner composition used in the invention may comprise ≥ 0 wt.-% to ≤ 0.05 wt.-%, preferably ≥ 0.0001 wt.-% to ≤ 0.04 wt.-%, more preferred ≥ 0.0003 wt.-% to ≤ 0.03 wt.-%, and most preferred of > 0.0005 wt.-% to ≤ 0.02 wt.-% of at least one corrosion inhibitor, preferably benzotriazole, and most preferred at least one 1,2,3-benzotriazole; based on the total weight amount of the diluted disinfectant cleaner composition used in the invention.

It should be understood that the disinfectant cleaner composition used in the invention can be preferably free of a corrosion inhibitor.

### Sequestering agent

The disinfectant cleaner composition may in addition comprises at least one sequestering agent selected from the group of sodium gluconate, pentasodium salt of diethylenetriamine pentaacetic acid (DTPA), sodium glucoheptonate, salts of ethylene diamine tetraacetic acid (EDTA), salts of ethylene diamine tetraacetic acid, salts of hydroxyethyl ethylene diamine triacetic acid, salts of hydroxyethyl ethylene diamine triacetic acid, salts of nitrilotriacetic acid, salts of nitrilotriacetic acid (NTA), diethanolglycine sodium salt, ethanoldiglycine disodium salt, salts of hydroxymonocarboxylic acid compounds, salts of hydroxydicarboxylic acid compounds, salts of amine containing carboxylic acids, terasodium N,N-bis(carboxylatomethyl)-L-glutamate (GLDA), hydroxyethylethylene-diaminetriacetate (HEDTA), or any combination thereof, and more preferred methylglycinediacetate (MGDA).

The disinfectant composition, preferably in form of a concentrate, may comprises ≥ 0 wt.-% to ≤ 1 wt.-%, preferably ≥ 0.05 wt.-% to ≤ 0.8 wt.-%, more preferred ≥ 0.07 wt.-% to ≤ 0.6 wt.-%, and most preferred of ≥ 0.1 wt.-% to ≤ 0.5 wt.-% of at least one sequestering agent, preferably methylglycinediacetate (MGDA); based on the total weight amount of the disinfectant cleaner composition used in the invention, preferably in form of a concentrate.

A diluted disinfectant cleaner composition used in the invention may comprise ≥ 0 wt.-% to ≤ 0.05 wt.-%, preferably ≥ 0.0001 wt.-% to ≤ 0.04 wt.-%, more preferred ≥ 0.0003 wt.-% to ≤ 0.03 wt.-%, and most preferred of > 0.0005 wt.-% to ≤ 0.02 wt.-% of at least one sequestering agent, preferably methylglycinediacetate (MGDA); based on the total weight amount of the diluted disinfectant cleaner composition used in the invention.

It should be understood that the sequestering agent can include mixtures of different sequestering agents.

It should be understood that the disinfectant cleaner composition can be preferably free of a sequestering agent.

### Additional solvents

Suitable additional solvents include, water, alcohols, ethanol, isopropanol, 2-butoxy ethanol, 1-decanol, benzyl alcohol, glycerin, monoethanolamine, glycols, ethylene glycol, diethylene glycol, propylene glycol, butoxy diglycol, triethylene glycol, tetraethylene glycol, glycerin, propylene glycol, dipropylene glycol, hexylene glycol, glycol ethers, esters, or combinations thereof. Suitable alcohols include, ethanol, isopropanol, 2-butoxy ethanol, 1-decanol, benzyl alcohol, glycerin, monoethanolamine, or any combination thereof, and preferably the solvent is water.

The disinfectant composition, preferably in form of a concentrate, may comprises ≥ 0 wt.-% to ≤ 92 wt.-%, preferably ≥ 20 wt.-% to ≤ 80 wt.-%, more preferred ≥ 50 wt.-% to ≤ 75 wt.-%, and further more preferred ≥ 55 wt.-% to ≤ 70 wt.-%, preferably the solvent is add. to 100 wt.-%, and most preferably the solvent is water; based on the total weight amount of the disinfectant cleaner composition used in the invention, preferably in form of a concentrate.

A diluted disinfectant cleaner composition used in the invention may comprise of at least one solvent of ≥ 0 wt.-% to < 100 wt.-%, preferably ≥ 0.05 wt.-% to ≤ 99,5 wt.-%, more preferred ≥ 0.05 wt.-% to ≤ 99 wt.-%, and further more preferred ≥ 0.07 wt.-% to ≤ 99 wt.-%, preferably the solvent is add. to 100 wt.-%, and most preferably the solvent is water; based on the total weight amount of the diluted disinfectant cleaner composition used in the invention.

### Concentrate

The disinfectant cleaner composition can be presented in a liquid concentrated form. The source of alkalinity and addition of the solvent, preferably water, are provided so that the concentrated, preferably aqueous, liquid composition of the disinfectant cleaner composition according to the present invention may have a pH in the range of > 7 pH to ≤ 14 pH, preferably is from ≥ 9 pH to ≤ 13 pH, and more preferred is from ≥ 10 pH to ≤ 12 pH.

According to one embodiment, the disinfectant cleaner composition used in the invention, preferably in form of a concentrate, may comprise:
- ≥ 4 wt.-% to ≤ 11 wt.-%, preferably ≥ 5 wt.-% to ≤ 10 wt.-%, more preferred ≥ 6 wt.-% to ≤ 9 wt.-%, and most preferred of > 7 wt.-% to ≤ 8 wt.-% of at least one C8 to C18 N-alkyl dimethyl benzyl ammonium chloride, preferably a mixture of a N-alkyl(C10-C18)dimethyl benzyl ammonium chloride, further preferred N-alkyl(C10-C18)dimethyl benzyl ammonium chloride, further further preferred dodecyl dimethyl benzyl ammonium chloride and/or more preferred a N-alkyl(C10-C18)dimethyl benzyl ammonium chloride mixture; and/or mixtures thereof;
- ≥ 4 wt.-% to ≤ 12 wt.-%, preferably ≥ 5 wt.-% to ≤ 11 wt.-%, more preferred ≥ 6 wt.-% to ≤ 10 wt.-%, and most preferred of ≥ 7 wt.-% to ≤ 9 of at least one bis(3-aminopropyl) C8-C18 alkylamine, or any combination thereof, and most preferred bis(3-aminopropyl) dodecylamine;
- ≥ 0 wt.-% to ≤ 20 wt.-%, preferably ≥ 5 wt.-% to ≤ 15 wt.-%, more preferred ≥ 8 wt.-% to ≤ 12 wt.-%, and most preferred of ≥ 9 wt.-% to ≤ 11 wt.-% of at least one phenoxy alkanol, wherein the alkanol has from 1 to 6 carbon atoms or any combination thereof, and most preferred phenoxy ethanol;
- ≥ 0 wt.-% to ≤ 10 wt.-%, preferably ≥ 1 wt.-% to ≤ 8 wt.-%, more preferred ≥ 2 wt.-% to ≤ 6 wt.-%, and most preferred of > 3 wt.-% to ≤ 5 wt.-% of at least one C4 to C18 alkyl polyglycoside or any combination thereof, preferably at least one C6 to C18 alkyl polyglycoside and more preferred a mixture of C8 to C16 alkyl polyglycosides;
- ≥ 0 wt.-% to ≤ 1 wt.-%, preferably ≥ 0.05 wt.-% to ≤ 0.8 wt.-%, more preferred ≥ 0.1 wt.-% to ≤ 0.6 wt.-%, and most preferred of ≥ 0.15 wt.-% to ≤ 0.4 wt.-% of at least one corrosion inhibitor, preferably benzotriazole, and most preferred at least one 1,2,3-benzotriazole;
- ≥ 0 wt.-% to ≤ 1 wt.-%, preferably ≥ 0.05 wt.-% to ≤ 0.8 wt.-%, more preferred ≥ 0.07 wt.-% to ≤ 0.6 wt.-%, and most preferred of ≥ 0.1 wt.-% to ≤ 0.5 wt.-% of at least one sequestering agent, preferably methylglycinediacetate (MGDA);
- ≥ 0 wt.-% to ≤ 10 wt.-%, preferably ≥ 0.1 wt.-% to ≤ 8 wt.-%, more preferred ≥ 0.5 wt.-% to ≤ 5 wt.-%, and most preferred of > 1 wt.-% to ≤ 3 wt.-% of at least one alkali source, and more preferred ethanolamine;
- a solvent of ≥ 0 wt.-% to ≤ 92 wt.-%, preferably ≥ 20 wt.-% to ≤ 80 wt.-%, more preferred ≥ 50 wt.-% to ≤ 75 wt.-%, and further more preferred ≥ 55 wt.-% to ≤ 70 wt.-%, preferably the solvent is add. to 100 wt.-%, and most preferably the solvent is water;
wherein
the weight.-% of the components are based on the total weight of disinfectant cleaner composition and the weight.-% of all components of the composition are select so that it does not exceed 100 wt.-%.

### Ready-to-use composition

The disinfectant cleaner composition used in the invention can be present in form of a diluted or so called "ready-to-use" composition. The source of alkalinity and addition of the solvent, preferably water, are provided so that the diluted, preferably aqueous, liquid composition of the disinfectant cleaner composition according to the present invention may have a pH in the range of > 7 pH to ≤ 12 pH, preferably is from ≥ 7.5 pH to ≤ 11,5 pH, and more preferred is from ≥ 9 pH to ≤ 11,0 pH.

The concentrated disinfectant cleaner composition can be diluted with a at least one solvent, preferably water, by a factor of 10 to 1000, preferably 20 to 500 and further preferred 25 to 400 to obtain the diluted disinfectant cleaner composition used in the invention.

According to one aspect, the diluted disinfectant cleaner composition (ready-to-use) can be diluted with a solvent, preferably water, to a 0.25% to 4.0% solution from a concentrated disinfectant composition.

It will be appreciated that the actual concentration of components in a composition used in the invention will depend on the intended use of that composition. For disinfecting uses, such as cleaning of hospital wards and equipment to help prevent the spread of disease such as Norovirus, Adenovirus and Polyomavirus, higher concentrations are required than for certain sanitizing applications.

According to one embodiment, the diluted disinfectant cleaner composition may comprise:
- ≥ 0.005 wt.-% to ≤ 0.6 wt.-%, preferably ≥ 0.01 wt.-% to ≤ 0.5 wt.-%, more preferred ≥ 0.015 wt.-% to ≤ 0.4 wt.-%, and most preferred of > 0.015 wt.-% to ≤ 0.3 wt.-% of at least one at least one N-alkyl dimethyl benzyl ammonium chloride, wherein said alkyl has from 8 to 18 carbon atoms, can be selected from the group of at least one alkyl(C14) dimethyl benzyl ammonium chloride, alkyl(C16) dimethyl benzyl ammonium chloride, alkyl(C18) dimethyl benzyl ammonium chloride, a mixture of N-alkyl(C8-C18) dimethyl benzyl ammonium chloride, or a mixture of N-alkyl(C10-C18) dimethyl benzyl ammonium chloride, or any combination thereof; and more preferred a N-alkyl(C10-C18) dimethyl benzyl ammonium chloride mixture, further further preferred dodecyl dimethyl **benzyl** ammonium chloride and most preferred a N-alkyl(C10-C18) dimethyl benzyl ammonium chloride mixture, wherein the weight amount, based on the N-alkyl(C10-C18)dimethyl benzyl ammonium chloride mixture, of the N-dodecyl dimethyl benzyl ammonium chloride (C12) is > than N-quatrodecyl dimethyl benzyl ammonium chloride (C14) is > than N-hexadecyl dimethyl benzyl ammonium chloride (C16) is > than N-decyl dimethyl benzyl ammonium chloride (C10) > than N-octadecyl dimethyl benzyl ammonium chloride (C18) and/or mixtures thereof;
- ≥ 0.01 wt.-% to ≤ 0.6 wt.-%, preferably ≥ 0.015 wt.-% to ≤ 0.5 wt.-%, more preferred ≥ 0.02 wt.-% to ≤ 0.45 wt.-%, and most preferred of > 0.02 wt.-% to ≤ 0.4 wt.-% of at least one bis(3-aminopropyl) C8-C18 alkylamine, or any combination thereof, and most preferred bis(3-aminopropyl) dodecylamine;
- ≥ 0 wt.-% to ≤ 1 wt.-%, preferably ≥ 0.01 wt.-% to ≤ 0.8 wt.-%, more preferred ≥ 0.02 wt.-% to ≤ 0.6 wt.-%, and most preferred of ≥ 0.02 wt.-% to ≤ 0.5 wt.-% of at least one phenoxy alkanol, wherein the alkanol has from 1 to 6 carbon atoms or any combination thereof, and most preferred phenoxy ethanol;
- ≥ 0 wt.-% to ≤ 0.5 wt.-%, preferably ≥ 0.0025 wt.-% to ≤ 0.5 wt.-%, more preferred ≥ 0.005 wt.-% to ≤ 0.25 wt.-%, and most preferred of ≥ 0.01 wt.-% to ≤ 0.2 wt.-% of at least one C4 to C18 alkyl polyglycoside or any combination thereof, preferably at least one C6 to C18 alkyl polyglycoside and more preferred a mixture of C8 to C16 alkyl polyglycosides;
- ≥ 0 wt.-% to ≤ 0.05 wt.-%, preferably ≥ 0.0001 wt.-% to ≤ 0.04 wt.-%, more preferred ≥ 0.0003 wt.-% to ≤ 0.03 wt.-%, and most preferred of > 0.0005 wt.-% to ≤ 0.02 wt.-% of at least one corrosion inhibitor, preferably benzotriazole, and most preferred at least one 1,2,3-benzotriazole;
- ≥ 0 wt.-% to ≤ 0.05 wt.-%, preferably ≥ 0.0001 wt.-% to ≤ 0.04 wt.-%, more preferred ≥ 0.0003 wt.-% to ≤ 0.03 wt.-%, and most preferred of > 0.0005 wt.-% to ≤ 0.02 wt.-% of at least one sequestering agent, preferably methylglycinediacetate (MGDA);
- ≥ 0 wt.-% to ≤ 0.5 wt.-%, preferably ≥ 0.00025 wt.-% to ≤ 0.4 wt.-%, more preferred ≥ 0.001 wt.-% to ≤ 0.2 wt.-%, and most preferred of > 0.004 wt.-% to ≤ 0.1 wt.-% of at least one alkali source, and more preferred ethanolamine;
- a solvent of ≥ 0 wt.-% to < 100 wt.-%, preferably ≥ 0.05 wt.-% to ≤ 99,5 wt.-%, more preferred ≥ 0.05 wt.-% to ≤ 99 wt.-%, and further more preferred ≥ 0.07 wt.-% to ≤ 99 wt.-%, preferably the solvent is add. to 100 wt.-%, and most preferably the solvent is water; wherein
the weight.-% of the components are based on the total weight of disinfectant cleaner composition and the weight.-% of all components of the composition are select so that it does not exceed 100 wt.-%.

### Use of the disinfectant composition

The disinfectant cleaner is used for inactivating Adenovirus and Simianvirus 40. Preferably a disinfectant cleaner composition of the invention having a residual effect and tested in this manner will give a log reduction of at least 3.0, more preferably of at least 4.0.

The inactivation takes place, for example on surfaces in hospitals, industrial facilities and research laboratories, particularly selected from surfaces of instruments employed in medical, dental, and pharmaceutical procedures, surfaces of equipment, the inanimate patient environment, inanimate surfaces in operating theatres and other areas within healthcare, processing facilities or containers used in the food service, food processing, butchery, dairy, beverage, brewery, and pharmaceutical industries, work surfaces, walls, floors, ceilings, fermentation tanks, and fluid supply lines.

Using the disinfectant compositions can take the form of a concentrate that can be diluted and combined to provide a ready-to-use solution, and as a ready-to-use liquid composition that can be used to clean articles having a metal or plastic surface, such as surgical, medical, and dental instruments, including endoscopes.

Metal surfaces and/or plastic surfaces in need of disinfecting and cleaning are found in several locations. Exemplary locations include surgical instruments, medical instruments, and dental instruments, sinks, cookware, utensils, machine parts, vehicles, tanker trucks, vehicle wheels, work surfaces, tanks, immersion vessels, spray washers, and ultrasonic baths.

Metal surfaces that can be disinfected include iron-based metals such as iron, iron alloys, e. g. steel, tin, aluminum, copper, tungsten, titanium, molybdenum, for example. The structure of the metal surface to be disinfected can vary widely. Thus, the metal surface and/or plastic surface can be as a metal and/or plastic part of complex configuration, sheeting, coils, rolls, bars, rods, plates, disks.

More preferred is the use of the disinfectant cleaner composition, in particular the ready-to-use composition to disinfect metal and/or plastic articles, especially metal instruments, plastic instruments, instruments with a plastic surface and/or instruments with a metal surface, surfaces of equipment, the inanimate patient environment, inanimate surfaces in operating theatres and other areas within healthcare.

The disinfectant composition, preferably the ready-to-use-composition, can be applied to a surface by wiping the treated surface with a saturated cloth, mop, sponge or other suitable delivery mechanism. The composition can also be applied by spraying and/or flooding the surface with the disinfectant composition or by immersion of items in the use solution.

The disinfectant cleaner composition is maybe suitable for a variety of consumer applications. Examples of the formulations of the invention include surface cleaners such as those intended for use in bathrooms, kitchens, living areas hard floor cleaners carpet cleaners furniture cleaners, glass/mirror cleaners; toilet care products including solid toilet cleaners such as rim devices and those designed to be placed in the cistern liquid toilet cleaners excluding those comprising hypochlorite bleaches: dishwashing products such as washing up liquids and preparations from dishwashing machines such as dishwashing ≥ 7 pH to ≤ 14 pH, preferably is from ≥ 9 pH to ≤ 13 pH, and more preferred is from ≥ 10 pH to ≤ 12 pH liquids; laundry products such as liquid detergents and fabric conditioners and "2 in 1" products comprising detergent and fabric conditioner; cleaning products intended for use outdoors such as those for cleaning for wood, stone, concrete or plastics, for example patio cleaner, garden furniture cleaners/treatments, BBQ cleaners, wall and fence cleaners/ treatments, plant sprays such as those intended to remove insects such as aphides from plants; food sprays, such as those suitable for use in food preservation; personal care products such as bath and shower products; soaps, including liquid soaps, hand sanitizers, deodorants and antiperspirants, hair care products including shampoos, for example anti-scalp odor shampoos, shampoos for the control of head lice eggs and anti- dandruff shampoos, hair conditioners, hair styling products such as hair mousses, gels and sprays, skin care products such as shaving products, cosmetics and products for hair removal; baby products including baby cleaning and cleansing products such as baby bath, soaps, wipes, moisturizers, nappy rash cream, products for cleaning surfaces that have regular & high incidence of infant & baby contact; first aid products and products for treating ailments and illnesses, including products for the topical treatment and/or prevention of minor infections such as athletes foot, spot/acne prevention/treatment products; foot hygiene products, including those for use on the foot and those for the toot ware, particularly sports foot wear; products for cleaning and/or deodorizing vehicles such as cars.
The invention also provides a process for making the compositions. The process comprises the steps of mixing at least part of at least one quaternary ammonium chloride of N-alkyl dimethyl benzyl ammonium chloride, wherein said alkyl has from 8 to 18 carbon atoms, further preferred dodecyl dimethyl benzyl ammonium chloride, and adding the at least one bis(3-aminopropyl) alkylamine, wherein said alkyl has from 6 to 18 carbon atoms, preferably bis(3-aminopropyl) dodecylamine; wherein
the weight-% ratio of a) a quaternary ammonium chloride of N-alkyl dimethyl benzyl ammonium chloride, wherein said alkyl has from 8 to 18 carbon atoms, further preferred dodecyl dimethyl benzyl ammonium chloride, to b) bis(3-aminopropyl) alkylamine, wherein said alkyl has from 6 to 18 carbon atoms, preferably bis(3-aminopropyl) dodecylamine, is in the range of > 0.1 : 1 to < 1 : 1, in the appropriate amounts to achieve the synergy of the two, and any further components, such as phenoxy ethanol, solvent, preferably water. and agitating the resulting mixture until a homogeny solution is formed. Typically, the process to produce the compositions of the invention is carried out at room temperature with stirring. The present invention provides compositions obtainable by the process set out above. The compositions of the invention may be prepared in a concentrated form (i.e. with no solvent or little solvent) and diluted with a solvent, preferably water when used to the diluted disinfectant cleaner solution.

The following are non-limiting examples of table 2 are comparative compositions and are intended for purposes of illustration only.

### I. Concentrated disinfectant cleaner composition

**Table 1**

| Concentrated disinfectant cleaner composition | | | | | | | |
|---|---|---|---|---|---|---|---|
| Components wt.-% | C1 | C2 | C3 | V4 | V5 | V6 | V7 |
| Benzalkoniumchlorid*¹ | 22.0 | 7.5 | 22.5 | 7.5 | 7.5 | 7.5 | 7.5 |
| N-(3-aminopropyl)-N-dodecylpro-pane-1,3-diamine | 0 | 4.0 | 8.0 | 8.0 | 8.0 | 8.0 | 8.0 |
| Phenoxyethanol | 17 | 0 | 0 | 0 | 10 | 10 | 10 |
| Monoethanolamin | 0 | 0 | 0 | 0 | 0 | 2 | 2 |
| MGDA *² | 0 | 0 | 0 | 0 | 0 | 0 | 0.25 |
| Benzotriazol | 0 | 0 | 0 | 0 | 0 | 0 | 0.2 |
| C8 to C16 alkyl polyglycoside | 0 | 0 | 0 | 0 | 0 | 0 | 4 |
| H₂O | add. 100 | add. 100 | add. 100 | add. 100 | add. 100 | add. 100 | add. 100 |

| | | | | | | | |
|---|---|---|---|---|---|---|---|
| *¹ = Alkyl dimethyl benzyl ammonium chloride (C10 1,5%, C12 64,73%, C14 22-29%, C16 2-6%) *² = Methylglycinediacetate (MGDA) | | | | | | | |

### EXAMPLES

### EXAMPLE 1

The concentrated disinfectant cleaner compositions C1, C2, C3, and V4,V5, V6 and V7 were each diluted with water to a 0.5%, 1%, 2%, 3% concentrated diluted disinfectant cleaner composition E1(C1), E2(C2), E3(C3), E4(V4), E5(V5), E6(V6) to E7(V7) respectively.

V4, V5, V6 and V7 as well as E4, E5, E6 and E7 are examples of the invention.

C1, C2 and C3 as well as E1, E2 and E3 are comparative examples.

The European Standard EN 14348 defines the test method (phase 2/ step 1) and the requirements for the mycobacteria activity for disinfectants used in medical area including surface and instruments disinfectants.

The mycobacterial activity is defined as the capability to reduce the viable counts of mycobacteria test strains by a factor of minimum lg 4 within 60 minutes at 20° C under the influence of either clean or dirty conditions.

The tests were run with *Mycobacterium terrae* and *Mycobacterium avium* at 20° C with contact times of 60 and 120 minutes under clean and dirty conditions.

**Table 2**

| Activity against Mycobacterium terrae and Mycobacterium avium for illustration only | | | | | |
|---|---|---|---|---|---|
| disinfectant cleaner composition | | *Mycobacterium terrae* | | *Mycobacterium avium* | |
| | | clean cond. | dirty cond. | clean cond. | dirty cond. |
| 0.5% | E1 | <1.5 lg / 60 min | <1.87 lg / 60 min | <1.87 lg / 60 min | <1.79 lg/ 60 min |
| 1 % | E1 | <1.5 lg / 60 min | <1.87 lg / 60 min | <1.87 lg / 60 min | <1.79 lg/ 60 min |
| 2% | E1 | <1.5 lg / 60 min | <1.87 lg / 60 min | <1.87 lg / 60 min | <1.79 lg/ 60 min |
| 3% | E1 | <1.5 lg / 60 min | <1.87 lg / 60 min | <1.87 lg / 60 min | <1.79 lg/ 60 min |
| 0.5% | E2 | <1.5 lg / 60 min | <1.87 lg / 60 min | <1.87 lg / 60 min | <1.79 lg/ 60 min |
| 1 % | E2 | <1.5 lg / 60 min | <1.87 lg / 60 min | <1.87 lg / 60 min | <1.79 lg/ 60 min |
| 2% | E2 | <1.5 lg / 60 min | <1.87 lg / 60 min | <1.87 lg / 60 min | <1.79 lg/ 60 min |
| 3% | E2 | 2.09 lg / 60 min | 3.0 lg / 60 min | 3.72 lg / 60 min | 2.89 lg / 60 min |
| 0.5% | E3 | <1.5 lg / 60 min | <1.87 lg / 60 min | 2.9 lg / 60 min | 2.0 lg / 60 min |
| 1 % | E3 | <1.5 lg / 60 min | <1.87 lg / 60 min | 3.55 lg / 60 min | 2.29 lg / 60 min |
| 2% | E3 | <1.5 lg / 60 min | <1.87 lg / 60 min | 3.71 lg / 60 min | 3.99 lg / 60 min |
| 3% | E3 | 2.3 lg / 60 min | <1.87 lg / 60 min | 4.42 lg / 60 min | 4.14 lg / 60 min |
| 0.5% | E4 | | 3.56 lg / 60 min | | >6.54 lg / 60 min |
| | | | 4.53 lg / 120 min | | |
| 1 % | E4 | | 4.98 lg / 60 min | | >6.54 lg / 60 min |
| | | | 6.53 lg / 120 min | | |
| 2% | E4 | | 5.22 lg / 60 min | | >6.54 lg / 60 min |
| | | | 6.32 lg / 120 min | | |
| 3% | E4 | | 5.32 lg / 60 min | | >6.54 lg / 60 min |
| 0.5% | E5 | | 3.82 lg / 60 min | | >6.54 lg / 60 min |
| | | | 6.2 lg / 120 min | | |
| 1 % | E5 | | 4.48 lg / 60 min | | >6.54 lg / 60 min |
| | | | >6.53 lg / 120 min | | |
| 2% | E5 | | 4.78 lg / 60 min | | >6.54 lg / 60 min |
| | | | >6.53 lg / 120 min | | |
| 3% | E5 | | 5.36 lg / 60 min | | >6.54 lg / 60 min |
| 0.5% | E6 | | 3.54 lg / 60 min | | >6.54 lg / 60 min |
| | | | >6.53 lg / 120 min | | |
| 1 % | E6 | | 4.72 lg / 60 min | | >6.54 lg / 60 min |
| | | | >6.53 lg / 120 min | | |
| 2% | E6 | | 4.83 lg / 60 min | | >6.54 lg / 60 min |
| | | | >6.53 lg / 120 min | | |
| 3% | E6 | | 5.27 lg / 60 min | | >6.54 lg / 60 min |
| 0.5% | E7 | | 3.66 lg / 60 min | | >6.54 lg / 60 min |
| | | | 6.1 lg / 120 min | | |
| 1 % | E7 | | 4.56 lg / 60 min | | >6.54 lg / 60 min |
| | | | >6.53 lg / 120 min | | |
| 2% | E7 | | 4.93 lg / 60 min | | >6.54 lg / 60 min |
| | | | >6.53 lg / 120 min | | |
| 3% | E7 | | 5.53 lg / 60 min | | >6.54 lg / 60 min |

### EXAMPLE 2

The concentrated disinfectant cleaner compositions C1, C2, C3 and, V4, V5, V6 and V7 were each diluted with water to a 0.5%, 1%, 2%, 3% concentrated diluted disinfectant cleaner composition E1(C1), E2(C2), E3(C3), E4(V4), E5(V5), E6(V6) to E7(V7) respectively.

The guideline of the German Society for the Prevention of Viral Disease (DVV 2008) defines the test method (phase 2/ step 1) and the requirements for the virucidal activity for disinfect-tants used in medical area including instruments disinfectants.

The virucidal activity is defined as the capability to reduce the infectivity of test viruses by a factor of minimum lg 4 within the corresponding contact time at 20° C with and without the addition of an organic soil.

The tests were run with Adenovirus and Simianvirus 40 at 20° C with contact times of 60 and 120 minutes with and without organic soil.

**Table 3**

| Activity against Adenovirus and Simianvirus 40 | | | | | |
|---|---|---|---|---|---|
| disinfectant cleaner composition | | *Adenovirus* | | *SV40* | |
| | | Without organic soil | with organic soil | Without organic soil | with organic soil |
| 0.5% | E1 | | | 3.25 lg / 60 min | 2.00 lg / 60 min |
| 1 % | E1 | | | 2.63 lg / 60 min | 3.88 lg / 60 min |
| 2% | E1 | | >3.75 lg / 120 min | | |
| 3% | E1 | 2.19 lg / 120 min | | | |
| 0.5% | E2 | | | | |
| 1 % | E2 | | | | |
| 2% | E2 | | | | |
| 3% | E2 | >4.19 lg / 60 min | 2.87 lg / 60 min | >4.38 / 60 min | >4.63 / 60 min |
| 0.5% | E3 | 1.94 lg / 60 min | | >4.25 / 60 min | 2.25 lg / 60 min |
| 1 % | E3 | >4.0 lg / 60 min | | >4.25 / 60 min | >4.63 lg / 60 min |
| 2% | E3 | >4.0 lg / 60 min | >4.19 lg / 60 min | >4.25 / 60 min | >4.63 lg / 60 min |
| 3% | E3 | | | >4.25 / 60 min | >4.63 lg / 60 min |
| 0.5% | E4 | | 1.82 lg / 60 min | | 0.19 lg / 60 min |
| 1 % | E4 | | 2.0 lg / 60 min | | 3.07 lg / 60 min |
| 2% | E4 | | 3.0 lg / 60 min | | |
| 3% | E4 | | | | |
| 0.5% | E5 | | | | 0.57 lg / 60 min |
| 1 % | E5 | | 2.62 lg / 60 min | | 3.82 lg / 60 min |
| 2% | E5 | | >4.0 lg / 60 min | | >4.63 lg / 60 min |
| 3% | E5 | | | | |
| 0.5% | E6 | | | | 0.78 lg / 60 min |
| 1 % | E6 | | 2.72 lg / 60 min | | 3.98 lg / 60 min |
| 2% | E6 | | >4.0 lg / 60 min | | >4.63 lg / 60 min |
| 3% | E6 | | | | |
| 0.5% | E7 | | | | 0.87 lg / 60 min |
| 1 % | E7 | | 2.88 lg / 60 min | | 3.89 lg / 60 min |
| 2% | E7 | | >4.0 lg / 60 min | | >4.63 lg / 60 min |
| 3% | E7 | | | | |

The results of tables 2 and 3 clear demonstrate the improved mycobacterial activity of E4 to E7 compared to E1 to E3 as well as an improved virucidal effect in particular of E5 to E7 compared with E1 to E3 against Adenovirus and Simianvirus 40.

## Claims

1. Non-therapeutic use of a disinfectant cleaner composition for inactivating and/or reducing viruses on a hard and/or soft surface, wherein the viruses are selected from Adenovirus or Simianvirus 40, the composition comprising:
a) at least one quaternary ammonium chloride of N-alkyl dimethyl benzyl ammonium chloride, wherein said alkyl has from 8 to 18 carbon atoms
b) at least one bis(3-aminopropyl) alkylamine, wherein said alkyl has from 6 to 18 carbon atoms;
wherein the weight-% ratio of a) a quaternary ammonium chloride of N-alkyl dimethyl benzyl ammonium chloride, wherein said alkyl has from 8 to 18 carbon atoms to b) bis(3-aminopropyl) alkylamine, wherein said alkyl has from 6 to 18 carbon atoms, is in the range of > 0.1 : 1 to < 1 : 1.

2. The use of claim 1, wherein the non-therapeutic disinfectant cleaner composition comprises a phenoxy alkanol, wherein the alkanol has from 1 to 6 carbon atoms, or any combination thereof, and most preferred the phenoxy alkanol is phenoxy ethanol.

3. The use of claim 1 or 2, wherein the non-therapeutic disinfectant cleaner composition comprises in addition of at least one alkali source, preferably selected from the group comprising alkali metal hydroxides, alkali metal salts and/or amines and mixtures thereof, preferably triethanol amine, sodium hydroxide, potassium hydroxide, sodium carbonate, and/or sodium bicarbonate and mixtures thereof and more preferred ethanolamine.

4. The use of claims 1 to 3, wherein the non-therapeutic disinfectant cleaner composition comprises in addition of at least one nonionic surfactant, preferably at least one C4 to C18 alkyl polyglycoside or any combination thereof, preferably at least one C8 to C16 alkyl polyglycoside and more preferred a mixture of C8 to C16 alkyl polyglycosides.

5. The use of claims 1 to 4, wherein the non-therapeutic disinfectant cleaner composition comprises in addition of at least one corrosion inhibitor selected from the group comprising silicate, sodium silicate, sodium disilicate, calcium acetate, calcium chloride, calcium gluconate, calcium phosphate, calcium borate, calcium carbonate, calcium citrate, calcium lactate, calcium sulfate, calcium tartrate, benzotriazole, 1,2,3-benzotriazole, or any combination thereof, more preferred at least one benzotriazole, and most preferred at least one 1,2,3-benzotriazole.

6. The use of claims 1 to 5, wherein the non-therapeutic disinfectant cleaner composition comprises in addition of at least one sequestering agent selected from the group of sodium gluconate, pentasodium salt of diethylenetriamine pentaacetic acid (DTPA), sodium glucoheptonate, salts of ethylene diamine tetraacetic acid (EDTA), salts of ethylene diamine tetraacetic acid, salts of hydroxyethyl ethylene diamine triacetic acid, salts of hydroxyethyl ethylene diamine triacetic acid, salts of nitrilotriacetic acid, salts of nitrilotriacetic acid (NTA), diethanolglycine sodium salt , ethanoldiglycine disodium salt, salts of hydroxymonocarboxylic acid compounds, salts of hydroxydicarboxylic acid compounds, salts of amine containing carboxylic acids, terasodium N,N-bis(carboxylatomethyl)-L-glutamate (GLDA), hydroxyethylethylene-diaminetriacetate (HEDTA), or any combination thereof, and more preferred methylglycinediacetate (MGDA).

7. The use of claims 1 to 6, wherein the non-therapeutic disinfectant cleaner composition comprises in addition of at least one solvent selected from the group comprising water, alcohols, ethanol, isopropanol, 2-butoxy ethanol, 1-decanol, benzyl alcohol, glycerin, monoethanolamine, glycols, ethylene glycol, diethylene glycol, propylene glycol, butoxy diglycol, triethylene glycol, tetraethylene glycol, glycerin, propylene glycol, dipropylene glycol, hexylene glycol, glycol ethers, esters, or combinations thereof. Suitable alcohols include ethanol, isopropanol, 2-butoxy ethanol, 1-decanol, benzyl alcohol, glycerin, monoethanolamine, or any combination thereof, and preferably the solvent is water.

8. The use of claims 1 to 7, wherein the quaternary ammonium chloride of N-alkyl dimethyl benzyl ammonium chloride is selected from the group of at least one alkyl(C14) dimethyl benzyl ammonium chloride, alkyl(C16) dimethyl benzyl ammonium chloride, alkyl(C18) dimethyl benzyl ammonium chloride, a mixture of N-alkyl(C8-C18) dimethyl benzyl ammonium chloride, or a mixture of N-alkyl(C10-C18) dimethyl benzyl ammonium chloride, or any combination thereof; and more preferred a N-alkyl(C10-C18) dimethyl benzyl ammonium chloride mixture, and most preferred a N-alkyl(C10-C18) dimethyl benzyl ammonium chloride mixture, wherein the weight amount, based on the N-alkyl(C10-C18)dimethyl benzyl ammonium chloride mixture, of the N-dodecyl dimethyl benzyl ammonium chloride (C12) is > than N-quatrodecyl dimethyl benzyl ammonium chloride (C14) is > than N-hexadecyl dimethyl benzyl ammonium chloride (C16) is > than N-decyl dimethyl benzyl ammonium chloride (C10) > than N-octadecyl dimethyl benzyl ammonium chloride (C18); and/or mixtures thereof.

9. The use of claims 1 to 8, wherein the bis(3-aminopropyl) alkylamine is selected from the group comprising a bis(3-aminopropyl) C6-C18-alkylamine, a bis(3-aminopropyl) octylamine, a bis(3-aminopropyl) decylamine, a bis(3-aminopropyl) dodecylamine, a bis(3-aminopropyl) quatrodecylamine, a bis(3-aminopropyl) hexadecylamine, a bis(3-aminopropyl) octadecylamine, or any combination thereof, and most preferred is a bis(3-aminopropyl) dodecylamine.

10. The use of claims 1 to 9, wherein the pH of the composition is from ≥ 7 pH to ≤ 14 pH, preferably is from ≥ 9 pH to ≤ 13 pH, and more preferred is from ≥ 10 pH to ≤ 12 pH.

11. The use of claims 1 to 10, , wherein the non-therapeutic disinfectant cleaner composition is in form of a concentrate comprising:
- ≥ 4 wt.-% to ≤ 11 wt.-%, preferably ≥ 5 wt.-% to ≤ 10 wt.-%, more preferred ≥ 6 wt.-% to ≤ 9 wt.-%, and most preferred of ≥ 7 wt.-% to ≤ 8 wt.-% of at least one C8 to C18 N-alkyl dimethyl benzyl ammonium chloride, preferably a mixture of a N-alkyl(C10-C18)dimethyl benzyl ammonium chloride, further preferred N-dodecyl dimethyl benzyl ammonium chloride, and/or mixtures thereof;
- ≥ 4 wt.-% to ≤ 12 wt.-%, preferably ≥ 5 wt.-% to ≤ 11 wt.-%, more preferred ≥ 6 wt.-% to ≤ 10 wt.-%, and most preferred of ≥ 7 wt.-% to ≤ 9 wt.-% of at least one bis(3-aminopropyl) C8-C18 alkylamine, or any combination thereof, and most preferred bis(3-aminopropyl) dodecylamine;
- ≥ 0 wt.-% to ≤ 20 wt.-%, preferably ≥ 5 wt.-% to ≤ 15 wt.-%, more preferred ≥ 8 wt.-% to ≤ 12 wt.-%, and most preferred of ≥ 9 wt.-% to ≤ 11 wt.-% of at least one phenoxy alkanol, wherein the alkanol has from 1 to 6 carbon atoms or any combination thereof, and most preferred phenoxy ethanol;
- ≥ 0 wt.-% to ≤ 10 wt.-%, preferably ≥ 1 wt.-% to ≤ 8 wt.-%, more preferred ≥ 2 wt.-% to ≤ 6 wt.-%, and most preferred of ≥ 3 wt.-% to ≤ 5 wt.-% of at least one C4 to C18 alkyl polyglycoside or any combination thereof, preferably at least one C6 to C18 alkyl polyglycoside and more preferred a mixture of C8 to C16 alkyl polyglycosides;
- ≥ 0 wt.-% to ≤ 1 wt.-%, preferably ≥ 0.05 wt.-% to ≤ 0.8 wt.-%, more preferred ≥ 0.1 wt.-% to ≤ 0.6 wt.-%, and most preferred of ≥ 0.15 wt.-% to ≤ 0.4 wt.-% of at least one corrosion inhibitor, preferably benzotriazole, and most preferred at least one 1,2,3-benzotriazole;
- ≥ 0 wt.-% to ≤ 1 wt.-%, preferably ≥ 0.05 wt.-% to ≤ 0.8 wt.-%, more preferred ≥ 0.07 wt.-% to ≤ 0.6 wt.-%, and most preferred of ≥ 0.1 wt.-% to ≤ 0.5 wt.-% of at least one sequestering agent, preferably methylglycinediacetate (MGDA);
- ≥ 0 wt.-% to ≤ 10 wt.-%, preferably ≥ 0.1 wt.-% to ≤ 8 wt.-%, more preferred ≥ 0.5 wt.-% to ≤ 5 wt.-%, and most preferred of ≥ 1 wt.-% to ≤ 3 wt.-% of at least one alkali source, and more preferred ethanolamine;
- a solvent of ≥ 0 wt.-% to ≤ 92 wt.-%, preferably ≥ 20 wt.-% to ≤ 80 wt.-%, more preferred ≥ 50 wt.-% to ≤ 75 wt.-%, and further more preferred ≥ 55 wt.-% to ≤ 70 wt.-%, preferably the solvent is add. to 100 wt.-%, and most preferably the solvent is water; wherein
the weight.-% of the components are based on the total weight of disinfectant cleaner composition and the weight.-% of all components of the composition are select so that it does not exceed 100 wt.-%.

12. The use of claims 1 to 11, wherein the non-therapeutic disinfectant cleaner composition is in form of a diluted composition, wherein the concentrated disinfectant cleaner composition is diluted with a at least one solvent, preferably water, by a factor of 10 to 1000, preferably 50 to 500 and further preferred 25 to 400.

13. The use of claims 1 to 12, wherein the non-therapeutic disinfectant cleaner composition is in form of a diluted composition, comprising:
- ≥ 0.005 wt.-% to ≤ 0.6 wt.-%, preferably ≥ 0.01 wt.-% to ≤ 0.5 wt.-%, more preferred ≥ 0.015 wt.-% to ≤ 0.4 wt.-%, and most preferred of ≥ 0.015 wt.-% to ≤ 0.3 wt.-% of at least one C8 to C18 N-alkyl dimethyl benzyl ammonium chloride, preferably a mixture of a N-alkyl(C10-C18)dimethyl benzyl ammonium chloride, further preferred N-dodecyl dimethyl benzyl ammonium chloride, and/or mixtures thereof;
- ≥ 0.01 wt.-% to ≤ 0.6 wt.-%, preferably ≥ 0.015 wt.-% to ≤ 0.5 wt.-%, more preferred ≥ 0.02 wt.-% to ≤ 0.45 wt.-%, and most preferred of ≥ 0.02 wt.-% to ≤ 0.4 wt.-% of at least one bis(3-aminopropyl) C8-C18 alkylamine, or any combination thereof, and most preferred bis(3-aminopropyl) dodecylamine;
- ≥ 0 wt.-% to ≤ 1 wt.-%, preferably ≥ 0.01 wt.-% to ≤ 0.8 wt.-%, more preferred ≥ 0.02 wt.-% to ≤ 0.6 wt.-%, and most preferred of ≥ 0.02 wt.-% to ≤ 0.5 wt.-% of at least one phenoxy alkanol, wherein the alkanol has from 1 to 6 carbon atoms or any combination thereof, and most preferred phenoxy ethanol;
- ≥ 0 wt.-% to ≤ 0.5 wt.-%, preferably ≥ 0.0025 wt.-% to ≤ 0.5 wt.-%, more preferred ≥ 0.005 wt.-% to ≤ 0.25 wt.-%, and most preferred of ≥ 0.01 wt.-% to ≤ 0.2 wt.-% of at least one C4 to C18 alkyl polyglycoside or any combination thereof, preferably at least one C6 to C18 alkyl polyglycoside and more preferred a mixture of C8 to C16 alkyl polyglycosides;
- ≥ 0 wt.-% to ≤ 0.05 wt.-%, preferably ≥ 0.0001 wt.-% to ≤ 0.04 wt.-%, more preferred ≥ 0.0003 wt.-% to ≤ 0.03 wt.-%, and most preferred of ≥ 0.0005 wt.-% to ≤ 0.02 wt.-% of at least one corrosion inhibitor, preferably benzotriazole, and most preferred at least one 1,2,3-benzotriazole;
- ≥ 0 wt.-% to ≤ 0.05 wt.-%, preferably ≥ 0.0001 wt.-% to ≤ 0.04 wt.-%, more preferred ≥ 0.0003 wt.-% to ≤ 0.03 wt.-%, and most preferred of ≥ 0.0005 wt.-% to ≤ 0.02 wt.-% of at least one sequestering agent, preferably methylglycinediacetate (MGDA);
- ≥ 0 wt.-% to ≤ 0.5 wt.-%, preferably ≥ 0.00025 wt.-% to ≤ 0.4 wt.-%, more preferred ≥ 0.001 wt.-% to ≤ 0.2 wt.-%, and most preferred of ≥ 0.004 wt.-% to ≤ 0.1 wt.-% of at least one alkali source, and more preferred ethanolamine;
- a solvent of ≥ 0 wt.-% to < 100 wt.-%, preferably ≥ 0.05 wt.-% to ≤ 99,5 wt.-%, more preferred ≥ 0.05 wt.-% to ≤ 99 wt.-%, and further more preferred ≥ 0.07 wt.-% to ≤ 99 wt.-%, preferably the solvent is add. to 100 wt.-%, and most preferably the solvent is water; wherein
the weight.-% of the components are based on the total weight of disinfectant cleaner composition and the weight.-% of all components of the composition are select so that it does not exceed 100 wt.-%.

## Patentansprüche

1. Nicht-therapeutische Verwendung einer desinfizierenden Reinigerzusammensetzung für ein Deaktivieren und/oder Reduzieren von Viren auf einer harten und/oder weichen Oberfläche, wobei die Viren aus Adenovirus oder Simianvirus 40 ausgewählt sind, die Zusammensetzung umfassend:
a) mindestens ein quaternäres Ammoniumchlorid aus N-Alkyldimethylbenzylammoniumchlorid, wobei das Alkyl von 8 bis 18 Kohlenstoffatome aufweist
b) mindestens ein Bis(3-aminopropyl)alkylamin, wobei das Alkyl von 6 bis 18 Kohlenstoffatome aufweist,
wobei das Gew.-%-Verhältnis von a) einem quaternären Ammoniumchlorid aus N-Alkyldimethylbenzylammoniumchlorid, wobei das Alkyl von 8 bis 18 Kohlenstoffatome aufweist, zu b) Bis(3-aminopropyl)alkylamin, wobei das Alkyl von 6 bis 18 Kohlenstoffatome aufweist, in dem Bereich von > 0.1 : 1 bis < 1 : 1 liegt.

2. Verwendung nach Anspruch 1, wobei die nicht-therapeutische desinfizierende Reinigerzusammensetzung ein Phenoxyalkanol umfasst, wobei das Alkanol von 1 bis 6 Kohlenstoffatome aufweist, oder eine beliebige Kombination davon, und am stärksten bevorzugt das Phenoxyalkanol Phenoxyethanol ist.

3. Verwendung nach Anspruch 1 oder 2, wobei die nicht-therapeutische desinfizierende Reinigerzusammensetzung zusätzlich mindestens eine Alkaliquelle umfasst, vorzugsweise ausgewählt aus der Gruppe, umfassend Alkalimetallhydroxide, Alkalimetallsalze und/oder Amine und Mischungen davon, vorzugsweise Triethanolamin, Natriumhydroxid, Kaliumhydroxid, Natriumcarbonat und/oder Natriumbicarbonat und Mischungen davon und stärker bevorzugt Ethanolamin.

4. Verwendung nach den Ansprüchen 1 bis 3, wobei die nicht-therapeutische desinfizierende Reinigerzusammensetzung zusätzlich mindestens ein nichtionisches Tensid umfasst, vorzugsweise mindestens ein C4 bis C18-Alkylpolyglycosid oder eine beliebige Kombination davon, vorzugsweise mindestens ein C8 bis C16-Alkylpolyglycosid und stärker bevorzugt eine Mischung von C8 bis C16-Alkylpolyglycosiden.

5. Verwendung nach den Ansprüchen 1 bis 4, wobei die nicht-therapeutische desinfizierende Reinigerzusammensetzung zusätzlich mindestens einen Korrosionsinhibitor umfasst, ausgewählt aus der Gruppe, umfassend Silikat, Natriumsilikat, Natriumdisilikat, Calciumacetat, Calciumchlorid, Calciumgluconat, Calciumphosphat, Calciumborat, Calciumcarbonat, Calciumcitrat, Calciumlactat, Calciumsulfat, Calciumtartrat, Benzotriazol, 1,2,3-Benzotriazol oder eine beliebige Kombination davon, stärker bevorzugt umfassend mindestens einen Benzotriazol und am stärksten bevorzugt mindestens ein 1,2,3-Benzotriazol.

6. Verwendung nach den Ansprüchen 1 bis 5, wobei die nicht-therapeutische desinfizierende Reinigerzusammensetzung zusätzlich mindestens ein Maskierungsmittel umfasst, ausgewählt aus der Gruppe, umfassend Natriumgluconat, Pentanatriumsalz von Diethylentriaminpentaessigsäure (DTPA), Natriumglucoheptonat, Salze von Ethylendiamintetraessigsäure (EDTA), Salze von Ethylendiamintetraessigsäure, Salze von Hydroxyethylethylendiamintriessigsäure, Salze von Hydroxyethylethylendiamintriessigsäure, Salze von Nitrilotriessigsäure, Salze von Nitrilotriessigsäure (NTA), Diethanolglycinnatriumsalz, Ethanoldiglycindinatriumsalz, Salze von Hydroxymonocarbonsäureverbindungen, Salze von Hydroxydicarbonsäureverbindungen, Salze von aminhaltigen Carbonsäuren, Tetranatrium-N,N-bis(carboxylatomethyl)-L-glutamat (GLDA), Hydroxyethylethylendiamintriacetat (HEDTA) oder eine beliebige Kombination davon und stärker bevorzugt Methylglycinediacetat (MGDA).

7. Verwendung nach den Ansprüchen 1 bis 6, wobei die nicht-therapeutische desinfizierende Reinigerzusammensetzung zusätzlich mindestens ein Lösungsmittel umfasst, ausgewählt aus der Gruppe, umfassend Wasser, Alkohole, Ethanol, Isopropanol, 2-Butoxyethanol, 1-Decanol, Benzylalkohol, Glycerin, Monoethanolamin, Glykole, Ethylenglykol, Diethylenglykol, Propylenglykol, Butoxydiglykol, Triethylenglykol, Tetraethylenglykol, Glycerin, Propylenglykol, Dipropylenglykol, Hexylenglykol, Glykolether, Ester oder Kombinationen davon. Geeignete Alkohole schließen Ethanol, Isopropanol, 2-Butoxyethanol, 1-Decanol, Benzylalkohol, Glycerin, Monoethanolamin oder eine beliebige Kombination davon ein, und wobei vorzugsweise das Lösungsmittel Wasser ist.

8. Verwendung nach den Ansprüchen 1 bis 7, wobei das quaternäre Ammoniumchlorid von N-Alkyldimethylbenzylammoniumchlorid ausgewählt ist aus der Gruppe von mindestens einem Alkyl(C14)dimethylbenzylammoniumchlorid, Alkyl(C16)dimethylbenzylammoniumchlorid, Alkyl(C18)dimethylbenzylammoniumchlorid, einer Mischung von N-Alkyl(C8-C18)dimethylbenzylammoniumchlorid oder einer Mischung von N-Alkyl(C10-C18)dimethylbenzylammoniumchlorid oder einer beliebigen Kombination davon; und stärker bevorzugt einer N-Alkyl(C10-C18)-Dimethylbenzylammoniumchlorid-Mischung und am stärksten bevorzugt einer N-Alkyl(C10-C18)-Dimethylbenzylammoniumchlorid-Mischung, wobei die Gewichtsmenge, basierend auf der N-Alkyl(C10-C18)-Dimethylbenzylammoniumchlorid-Mischung, des N-Dodecyldimethylbenzylammoniumchlorid (C12) > ist als N-Quatrodecyldimethylbenzylammoniumchlorid (C14) > ist als N-Hexadecyldimethylbenzylammoniumchlorid (C16) > ist als N-Decyldimethylbenzylammoniumchlorid (C10) > ist als N-Octadecyldimethylbenzylammoniumchlorid (C18); und/oder Mischungen davon.

9. Verwendung nach den Ansprüchen 1 bis 8, wobei das Bis(3-aminopropyl)alkylamin ausgewählt ist aus der Gruppe, umfassend ein Bis(3-aminopropyl)-C6-C18-alkylamin, ein Bis(3-aminopropyl)octylamin, ein Bis(3-aminopropyl)decylamin, ein Bis(3-aminopropyl)dodecylamin, ein Bis(3-aminopropyl)quatrodecylamin, ein Bis(3-aminopropyl)hexadecylamin, ein Bis(3-aminopropyl)octadecylamin oder eine beliebige Kombination davon und am stärksten bevorzugt ein Bis(3-aminopropyl) dodecylamin ist.

10. Verwendung nach den Ansprüchen 1 bis 9, wobei der pH-Wert der Zusammensetzung von ≥ 7 pH bis ≤ 14 pH, vorzugsweise von ≥ 9 pH bis ≤ 13 pH und stärker bevorzugt von ≥ 10 pH bis ≤ 12 pH beträgt.

11. Verwendung nach den Ansprüchen 1 bis 10, wobei die nicht-therapeutische desinfizierende Reinigerzusammensetzung in Form eines Konzentrats ist, umfassend:
- zu ≥ 4 Gew.-% bis ≤ 11 Gew.-%, vorzugsweise zu ≥ 5 Gew.-% bis ≤ 10 Gew.-%, stärker bevorzugt zu ≥ 6 Gew.-% bis ≤ 9 Gew.-% und am stärksten bevorzugt zu ≥ 7 Gew.-% bis ≤ 8 Gew.-% mindestens ein C8 bis C18-N-Alkyldimethylbenzylammoniumchlorid, vorzugsweise eine Mischung von einem N-Alkyl(C10-C18)dimethylbenzylammoniumchlorid, ferner bevorzugt N-Dodecyldimethylbenzylammoniumchlorid, und/oder Mischungen davon;
- zu ≥ 4 Gew.-% bis ≤ 12 Gew.-%, vorzugsweise zu ≥ 5 Gew.-% bis ≤ 11 Gew.-%, stärker bevorzugt zu ≥ 6 Gew.-% bis ≤ 10 Gew.-% und am stärksten bevorzugtest zu ≥ 7 Gew.-% bis ≤ 9 Gew.-% mindestens ein Bis(3-aminopropyl)C8-C18-alkylamin oder eine beliebige Kombination davon und am stärksten bevorzugt Bis(3-aminopropyl)dodecylamin;
- zu ≥ 0 Gew.-% bis ≤ 20 Gew.-%, vorzugsweise zu ≥ 5 Gew.-% bis ≤ 15 Gew.-%, stärker bevorzugt zu ≥ 8 Gew.-% bis ≤ 12 Gew.-% und am stärksten bevorzugt zu ≥ 9 Gew.-% bis ≤ 11 Gew.-% mindestens ein Phenoxyalkanol, wobei das Alkanol von 1 bis 6 Kohlenstoffatome aufweist, oder eine beliebige Kombination davon, und am stärksten bevorzugt Phenoxyethanol;
- zu ≥ 0 Gew.-% bis ≤ 10 Gew.-%, vorzugsweise zu ≥ 1 Gew.-% bis ≤ 8 Gew.-%, stärker bevorzugt zu ≥ 2 Gew.-% bis ≤ 6 Gew.-% und am stärksten bevorzugt zu ≥ 3 Gew.-% bis ≤ 5 Gew.-% mindestens ein C4 bis C18-Alkylpolyglycosid oder eine beliebige Kombination davon, vorzugsweise mindestens ein C6 bis C18-Alkylpolyglycosid und stärker bevorzugt eine Mischung von C8 bis C16-Alkylpolyglycosiden;
- zu ≥ 0 Gew.-% bis ≤ 1 Gew.-%, vorzugsweise zu ≥ 0.05 Gew.-% bis ≤ 0,8 Gew.-%, stärker bevorzugt zu ≥ 0,1 Gew.-% bis ≤ 0,6 Gew.-% und am stärksten bevorzugt zu ≥ 0,15 Gew.-% bis ≤ 0,4 Gew.-% mindestens einen Korrosionsinhibitor, vorzugsweise Benzotriazol und am stärksten bevorzugt mindestens ein 1,2,3-Benzotriazol;
- zu ≥ 0 Gew.-% bis ≤ 1 Gew.-%, vorzugsweise zu ≥ 0,05 Gew.-% bis ≤ 0,8 Gew.-%, stärker bevorzugt zu ≥ 0,07 Gew.-% bis ≤ 0,6 Gew.-% und am stärksten bevorzugt zu ≥ 0,1 Gew.-% bis ≤ 0,5 Gew.-% mindestens ein Maskierungsmittel, vorzugsweise Methylglycinediacetat (MGDA);
- zu ≥ 0 Gew.-% bis ≤ 10 Gew.-%, vorzugsweise zu ≥ 0,1 Gew.-% bis ≤ 8 Gew.-%, stärker bevorzugt zu ≥ 0,5 Gew.-% bis ≤ 5 Gew.-% und am stärksten bevorzugt zu ≥ 1 Gew.-% bis ≤ 3 Gew.-% mindestens eine Alkaliquelle und am stärksten bevorzugt Ethanolamin;
- ein Lösungsmittel zu ≥ 0 Gew.-% bis ≤ 92 Gew.-%, vorzugsweise zu ≥ 20 Gew.-% bis ≤ 80 Gew.-%, stärker bevorzugt zu ≥ 50 Gew.-% bis ≤ 75 Gew.-% und ferner stärker bevorzugt zu ≥ 55 Gew.-% bis ≤ 70 Gew.-%, vorzugsweise beträgt das Lösungsmittel 100 Gew.-% und am stärksten bevorzugt ist das Lösungsmittel Wasser; wobei
wobei die Gew.-% der Komponenten auf das Gesamtgewicht der desinfizierenden Reinigerzusammensetzung bezogen sind und die Gew.-% aller Komponenten der Zusammensetzung gewählt sind, sodass sie 100 Gew.-% nicht überschreiten.

12. Verwendung nach den Ansprüchen 1 bis 11, wobei die nicht-therapeutische desinfizierende Reinigerzusammensetzung in Form einer verdünnten Zusammensetzung vorliegt, wobei die konzentrierte desinfizierende Reinigerzusammensetzung mit mindestens einem Lösungsmittel, vorzugsweise Wasser, um einen Faktor von 10 bis 1000, vorzugsweise 50 bis 500 und ferner bevorzugt 25 bis 400 verdünnt ist.

13. Verwendung nach den Ansprüchen 1 bis 12, wobei die nicht-therapeutische desinfizierende Reinigerzusammensetzung in Form einer verdünnten Zusammensetzung vorliegt, umfassend:
- zu ≥ 0,005 Gew.-% bis ≤ 0,6 Gew.-%, vorzugsweise zu ≥ 0,01 Gew.-% bis ≤ 0,5 Gew.-%, stärker bevorzugt zu ≥ 0,015 Gew.-% bis ≤ 0,4 Gew.-% und am stärksten bevorzugt zu ≥ 0,015 Gew.-% bis ≤ 0,3 Gew.-% mindestens ein C8 bis C18-N-Alkyldimethylbenzylammoniumchlorid, vorzugsweise eine Mischung von einem N-Alkyl(C10-C18)dimethylbenzylammoniumchlorid, ferner bevorzugt N-Dodecyldimethylbenzylammoniumchlorid, und/oder Mischungen davon;
- zu ≥ 0,01 Gew.-% bis ≤ 0,6 Gew.-%, vorzugsweise zu ≥ 0,015 Gew.-% bis ≤ 0,5 Gew.-%, stärker bevorzugtzu ≥ 0,02 Gew.-% bis ≤ 0,45 Gew.-% und am stärksten bevorzugt zu ≥ 0,02 Gew.-% bis ≤ 0,4 Gew.-% mindestens ein Bis(3-aminopropyl)C8-C18-alkylamin oder eine beliebige Kombination davon und am stärksten bevorzugt Bis(3-aminopropyl)dodecylamin;
- zu ≥ 0 Gew.-% bis ≤ 1 Gew.-%, vorzugsweise zu ≥ 0,01 Gew.-% bis ≤ 0,8 Gew.-%, stärker bevorzugt zu ≥ 0,02 Gew.-% bis ≤ 0,6 Gew.-% und am stärksten bevorzugt zu ≥ 0,02 Gew.-% bis ≤ 0,5 Gew.-% mindestens ein Phenoxyalkanol, wobei das Alkanol von 1 bis 6 Kohlenstoffatome aufweist, oder eine beliebige Kombination davon, und am stärksten bevorzugt Phenoxyethanol;
- zu ≥ 0 Gew.-% bis ≤ 0,5 Gew.-%, vorzugsweise zu ≥ 0,0025 Gew.-% bis ≤ 0,5 Gew.-%, stärker bevorzugt zu ≥ 0,005 Gew.-% bis ≤ 0,25 Gew.-% und am stärksten bevorzugt zu ≥ 0,01 Gew.-% bis ≤ 0,2 Gew.-% mindestens ein C4-bis C18-Alkylpolyglycosid oder eine beliebige Kombination davon, vorzugsweise mindestens ein C6 bis C18-Alkylpolyglycosid und stärker bevorzugt eine Mischung von C8 bis C16-Alkylpolyglycosiden;
- zu ≥ 0 Gew.-% bis ≤ 0,05 Gew.-%, bevorzugt zu ≥ 0.0001 Gew.-% bis ≤ 0,04 Gew.-%, stärker bevorzugt zu ≥ 0,0003 Gew.-% bis ≤ 0,03 Gew.-% und am stärksten bevorzugt zu ≥ 0,0005 Gew.-% bis ≤ 0,02 Gew.-% mindestens einen Korrosionsinhibitor, vorzugsweise Benzotriazol und am stärksten bevorzugt mindestens ein 1,2,3-Benzotriazol;
- zu ≥ 0 Gew.-% bis ≤ 0,05 Gew.-%, vorzugsweise zu ≥ 0,0001 Gew.-% bis ≤ 0,04 Gew.-%, stärker bevorzugt zu ≥ 0,0003 Gew.-% bis ≤ 0,03 Gew.-% und am stärksten bevorzugt zu ≥ 0,0005 Gew.-% bis ≤ 0,02 Gew.-% mindestens ein Maskierungsmittel, vorzugsweise Methylglycinediacetate (MGDA);
- zu ≥ 0 Gew.-% bis ≤ 0,5 Gew.-%, bevorzugt zu ≥ 0,00025 Gew.-% bis ≤ 0,4 Gew.-%, stärker bevorzugt zu ≥ 0,001 Gew.-% bis ≤ 0,2 Gew.-% und am stärksten bevorzugt zu ≥ 0,004 Gew.-% bis ≤ 0,1 Gew.-% mindestens eine Alkaliquielle und stärker bevorzugt Ethanolamin;
- ein Lösungsmittel zu ≥ 0 Gew.-% bis < 100 Gew.-%, vorzugsweise zu ≥ 0,05 Gew.-% bis ≤ 99,5 Gew.-%, stärker bevorzugt zu ≥ 0,05 Gew.-% bis ≤ 99 Gew.-% und ferner stärker bevorzugt zu ≥ 0,07 Gew.-% bis ≤ 99 Gew.-%, vorzugsweise beträgt das Lösungsmittel 100 Gew.-% und am stärksten bevorzugt ist das Lösungsmittel Wasser; wobei
wobei die Gew.-% der Komponenten auf das Gesamtgewicht der desinfizierenden Reinigerzusammensetzung bezogen sind und die Gew.-% aller Komponenten der Zusammensetzung gewählt sind, sodass sie 100 Gew.-% nicht überschreiten.

## Revendications

1. Utilisation non thérapeutique d'une composition nettoyante désinfectante permettant d'inactiver et/ou de réduire les virus sur une surface dure et/ou molle, dans laquelle les virus sont choisis parmi les Adénovirus ou les Simianvirus 40, la composition comprenant :
a) au moins un chlorure d'ammonium quaternaire de chlorure de N-alkyle diméthyle benzyle ammonium, dans lequel ledit alkyle a 8 à 18 atomes de carbone
b) au moins une bis (3-aminopropyl) alkylamine, dans laquelle ledit alkyle a de 6 à 18 atomes de carbone ;
dans laquelle le rapport en pourcentage de poids a) d'un chlorure d'ammonium quaternaire de chlorure de N-alkyle diméthyle benzyle ammonium, dans lequel ledit alkyle a 8 à 18 atomes de carbone et de b) bis (3-aminopropyl) alkylamine, dans lequel ledit alkyle a 6 à 18 atomes de carbone, est compris entre > 0,1 : 1 à < 1 : 1.

2. Utilisation selon la revendication 1, dans laquelle la composition nettoyante désinfectante non thérapeutique comprend un phénoxy-alcanol, dans lequel l'alcanol a 1 à 6 atomes de carbone, ou une combinaison quelconque de ceux-ci, et le plus préférablement le phénoxy-alcanol est le phénoxy-éthanol.

3. Utilisation selon la revendication 1 ou 2, dans laquelle la composition nettoyante désinfectante non thérapeutique comprend en outre au moins une source alcaline, de préférence choisie parmi le groupe comprenant des hydroxydes de métaux alcalins, des sels de métaux alcalins et/ou des amines et des mélanges de ceux-ci, de préférence le triéthanol amine, l'hydroxyde de sodium, l'hydroxyde de potassium, le carbonate de sodium et/ou le bicarbonate de sodium et des mélanges de ceux-ci, et plus préférablement, l'éthanolamine.

4. Utilisation selon les revendications 1 à 3, dans laquelle la composition nettoyante désinfectante non thérapeutique comprend en outre au moins un agent tensioactif non ionique, de préférence au moins un polyglycoside d'alkyle en C4 à C18 ou une combinaison quelconque de ceux-ci, de préférence au moins un polyglycoside d'alkyle en C8 à C16 et, plus préférablement, un mélange de polyglycosides d'alkyle en C8 à C16.

5. Utilisation selon les revendications 1 à 4, dans laquelle la composition nettoyante désinfectante non thérapeutique comprend en outre au moins un inhibiteur de corrosion choisi parmi le groupe comprenant le silicate, le silicate de sodium, le disilicate de sodium, l'acétate de calcium, le chlorure de calcium, le gluconate de calcium, le phosphate de calcium, le borate de calcium, le carbonate de calcium, le citrate de calcium, le lactate de calcium, le sulfate de calcium, le tartrate de calcium, le benzotriazole, le 1,2,3-benzotriazole ou une combinaison quelconque de ceux-ci, plus préférablement au moins un benzotriazole et idéalement au moins un 1,2,3- benzotriazole.

6. Utilisation selon les revendications 1 à 5, dans laquelle la composition nettoyante désinfectante non thérapeutique comprend en outre au moins un agent séquestrant choisi parmi le groupe du gluconate de sodium, du sel pentasodique de l'acide diéthylènetriaminepentaacétique (DTPA), du glucoheptonate de sodium, des sels de l'acide éthylène diamine tétraacétique (EDTA), des sels de l'acide éthylènediamine tétraacétique, des sels de l'acide hydroxyéthyléthylènediaminetriacétique, des sels de l'acide hydroxyéthyléthylènediamine triacétique, des sels de l'acide nitrilotriacétique, des sels de l'acide nitrilotriacétique (NTA), du sel de sodium de diéthanolglycine, du sel de sodium d'éthanoldiglycine, des sels de composés d'acide hydroxymonocarboxyle, des sels de composés d'acide hydroxydicarboxylique, des sels d'amine contenant des acides carboxyliques, du N,N-bis(carboxylatométhyl)-L-glutamate de terasodium (GLDA), de l'hydroxyéthyléthylène-diaminetriacétate (HEDTA), ou une combinaison quelconque de ceux-ci et plus préférablement du méthylglycinediacétate (MGDA).

7. Utilisation selon les revendications 1 à 6, dans laquelle la composition nettoyante désinfectante non thérapeutique comprend en outre au moins un solvant choisi parmi le groupe comprenant l'eau, les alcools, l'éthanol, l'isopropanol, le 2-butoxyéthanol, le 1-décanol, l'alcool benzylique, la glycérine, la monoéthanolamine, les glycols, l'éthylène glycol, le diéthylène glycol, le propylène glycol, le butoxy diglycol, le triéthylène glycol, le tétraéthylène glycol, la glycérine, le propylène glycol, le dipropylène glycol, l'hexylène glycol, les éthers de glycol, les esters ou une combinaison de ceux-ci. Les alcools appropriés comportent de l'éthanol, de l'isopropanol, du 2-butoxyéthanol, du 1-décanol, alcool benzylique, de la glycérine, de la monoéthanolamine ou une combinaison quelconque de ceux-ci, et de préférence le solvant est de l'eau.

8. Utilisation selon les revendications 1 à 7, dans laquelle le chlorure d'ammonium quaternaire de chlorure de N-alkyldiméthylbenzylammonium est choisi parmi le groupe constitué par au moins le chlorure d'alkyle (C14) diméthylbenzylammonium, le chlorure d'alkyle (C16) diméthylbenzylammonium, le chlorure d'alkyle (C18) diméthylbenzylammonium, un mélange de chlorure de N-alkyl (C8 à C18) diméthylbenzylammonium, ou un mélange de chlorure de N-alkyl (C10 à C18) diméthylbenzylammonium, ou une combinaison quelconque de ceux-ci ; et, plus préférablemennt, un mélange de chlorure de N-alkyl (C10 - C18) diméthyl benzyl ammonium, et le plus préférablement mélange de chlorure de N-alkyl (C10 - C18) diméthyl benzyl ammonium, dans lequel la quantité en poids, en fonction du mélange de chlorure de N-alkyl (C10 - C18) diméthyl benzyl ammonium, du chlorure de N-dodécyl diméthyl benzyl ammonium (C12) est > au chlorure de N-quatrodécyl diméthyl benzyl ammonium (C14) est > au chlorure de N-hexadécyl diméthyl benzyl ammonium (CI6) est > au chlorure de N-décyl diméthyl benzyl ammonium (C10) > au chlorure de N-octadécyl diméthyl benzyl ammonium (C18) ; et/ou des mélanges de ceux-ci.

9. Utilisation selon les revendications 1 à 8, dans laquelle la bis (3-aminopropyl) alkylamine est choisie parmi le groupe comprenant une bis (3-aminopropyl) alkylamine en C6 à C18, une bis (3-aminopropyl) octylamine, une bis (3-aminopropyl) décylamine, une bis (3-l'aminopropyl) dodécylamine, une bis (3-aminopropyl) quatrodécylamine, une bis (3-aminopropyl) hexadécylamine, une bis (3-aminopropyl) octadécylamine, ou une combinaison quelconque de celles-ci et le plus préférablement est une bis (3-aminopropyl) dodécylamine.

10. Utilisation selon les revendications 1 à 9, dans laquelle le pH de la composition est compris entre ≥ 7 pH et ≤ 14 pH, de préférence entre ≥ 9 pH et ≤ 13 pH, et, plus préférablement de ≥ 10 pH à ≤ 12 pH.

11. Utilisation selon les revendications 1 à 10, dans laquelle la composition nettoyante désinfectante non thérapeutique se présente sous la forme d'un concentré comprenant :
- ≥ 4 % en poids à ≤ 11 % en poids, de préférence ≥ 5 % en poids à ≤ 10 % en poids, plus préférablement ≥ 6 % en poids à ≤ 9 % en poids, et le plus préférablement, de ≥ 7 % en poids à ≤ 8 % en poids d'au moins un chlorure de N-alkyl diméthyl benzyl ammonium en C8 à C18, de préférence un mélange de chlorure de N-alkyl (C10-C18) diméthyl benzyl ammonium, en outre préférablement, un chlorure de N-dodécyl diméthyl benzyl ammonium, et/ou des mélanges de ceux-ci ;
- ≥ 4 % en poids à ≤ 12 % en poids, de préférence ≥ 5 % en poids à ≤ 11 % en poids, plus préférablement ≥ 6 % en poids à ≤ 10 % en poids, et le plus préférablement, de ≥ 7 % en poids à ≤ 9 % en poids d'au moins une bis (3-aminopropyl) alkylamine en C8-C18, ou une combinaison quelconque de celles-ci, et le plus préférablement, la bis (3-aminopropyl) dodécylamine ;
- ≥ 0 % en poids à ≤ 20 % en poids, de préférence ≥ 5 % en poids à ≤ 15 % en poids, plus préférablement ≥ 8 % en poids à ≤ 12 % en poids, et le plus préférablement, de ≥ 9 % en poids à ≤ 11 % en poids d'au moins un phénoxy-alcanol, dans lequel l'alcanol a de 1 à 6 atomes de carbone ou une combinaison quelconque de ceux-ci, et le plus préférablement, le phénoxy éthanol ;
- ≥ 0 % en poids à ≤ 10 % en poids, de préférence ≥ 1 % en poids à ≤ 8 % en poids, plus préférablement ≥ 2 % en poids à ≤ 6 % en poids, et le plus préférablement, de ≥ 3 % en poids à ≤ 5 % en poids d'au moins un polyglycoside d'alkyle en C4 à C18 ou une combinaison quelconque de ceux-ci, de préférence au moins un polyglycoside d'alkyle en C6 à C18 et, plus préférablement un mélange de polyglycosides d'alkyle en C8 à C16 ;
- ≥ 0 % en poids à ≤ 1 % en poids, de préférence ≥ 0,05 % en poids à ≤ 0,8 % en poids, plus préférablement ≥ 0,1 % en poids à ≤ 0,6 % en poids, et le plus préférablement ≥ 0,15 % en poids à ≤ 0,4 % en poids d'au moins un inhibiteur de corrosion, de préférence le benzotriazole, et le plus préférablement au moins un 1,2,3-benzotriazole ;
- ≥ 0 % en poids à ≤ 1 % en poids, de préférence ≥ 0,05 % en poids à ≤ 0,8 % en poids, plus préférablement ≥ 0,07 % en poids à ≤ 0,6 % en poids, et le plus préférablement ≥ 0,1 % en poids à ≤ 0,5 % en poids d'au moins un agent séquestrant, de préférence le méthylglycinediacétate (MGDA) ;
- ≥ 0 % en poids à ≤ 10 % en poids, de préférence ≥ 0,1 % en poids à ≤ 8 % en poids, plus préférablement ≥ 0,5 % en poids à ≤ 5 % en poids, et le plus préférablement ≥ 1 % en poids à ≤ 3 % en poids d'au moins une source alcaline, et plus préférablement d'éthanolamine ;
- un solvant ≥ 0 % en poids à ≤ 92 % en poids, de préférence ≥ 20 % en poids à ≤ 80 % en poids, plus préférablement ≥ 50 % en poids à ≤ 75 % en poids, et en outre plus préférablement ≥ 55 % en poids à ≤ 70 % en poids, de préférence le solvant est ajouté à 100 % en poids, et le plus préférablement le solvant est de l'eau ; dans lequel
le pourcentage en poids des composants est basé sur le poids total de la composition nettoyante désinfectante et le pourcentage en poids de tous les composants de la composition est choisi de sorte qu'il ne dépasse pas 100 % en poids.

12. Utilisation selon les revendications 1 à 11, dans laquelle la composition nettoyante désinfectante non thérapeutique se présente sous la forme d'une composition diluée, dans laquelle la composition nettoyante désinfectante concentrée est diluée avec au moins un solvant, de préférence de l'eau, d'un facteur de 10 à 1 000, de préférence de 50 à 500 et préférablement en outre de 25 à 400.

13. Utilisation selon les revendications 1 à 12, dans laquelle la composition de nettoyage désinfectante non thérapeutique se présente sous la forme d'une composition diluée, comprenant :
- ≥ 0,005 % en poids à ≤ 0,6 % en poids, de préférence ≥ 0,01 % en poids à ≤ 0,5 % en poids, plus préférablement ≥ 0,015 % en poids à ≤ 0,4 % en poids, et le plus préférablement ≥ 0,015 % en poids à ≤ 0,3 % en poids d'au moins un chlorure de **N-**alkyl diméthyl benzyl ammonium en C8 à C18, de préférence un mélange d'un **chlorure** de N-alkyl (C10- C18) diméthyl benzyl ammonium, préférablement en outre, un chlorure N-dodécyl diméthyl benzyl ammonium, et/ou des mélanges de ceux-ci ;
- ≥ 0,01 % en poids à ≤ 0,6 % en poids, de préférence ≥ 0,015 % en poids à ≤ 0,5 % en poids, plus préférablement ≥ 0,02 % en poids à ≤ 0,45 % en poids, et le plus préférablement ≥ 0,02 % en poids à ≤ 0,4 % en poids d'au moins une bis (3-aminopropyl) alkylamine en C8-C18, ou une combinaison quelconque de celles-ci, et le plus préférablement une bis (3-aminopropyl) dodécylamine ;
- ≥ 0 % en poids à ≤ 1 % en poids, de préférence ≥ 0,01 % en poids à ≤ 0,8 % en poids, plus préférablement ≥ 0,02 % en poids à ≤ 0,6 % en poids, et le plus préférablement de ≥ 0,02 % en poids à ≤ 0,5 % en poids d'au moins un phénoxy-alcanol, dans lequel l'alcanol a de 1 à 6 atomes de carbone ou toute combinaison de ceux-ci, et le plus préférablement du phénoxy-éthanol ;
- ≥ 0 % en poids à ≤ 0,5 % en poids, de préférence ≥ 0,0025 % en poids à ≤ 0,5 % en poids, plus préférablement ≥ 0,005 % en poids à ≤ 0,25 % en poids, et le plus préférablement de ≥ 0,01 % en poids à ≤ 0,2 % en poids d'au moins un polyglycoside d'alkyle en C4 à C18 ou toute combinaison de ceux-ci, de préférence au moins un polyglycoside d'alkyle en C6 à C18 et plus préférablement un mélange de polyglycosides d'alkyle en C8 à C16 ;
- ≥ 0 % en poids à ≤ 0,05 % en poids, de préférence ≥ 0,0001 % en poids à ≤ 0,04 % en poids, plus préférablement ≥ 0,0003 % en poids à ≤ 0,03 % en poids, et le plus préférablement ≥ 0,0005 % en poids à ≤ 0,02 % en poids d'au moins un inhibiteur de corrosion, de préférence le benzotriazole, et le plus préférablement, au moins un 1,2,3-benzotriazole
- ≥ 0 % en poids à ≤ 0,05 % en poids, de préférence ≥ 0,0001 % en poids à ≤ 0,04 % en poids, plus préférablement ≥ 0,0003 % en poids à ≤ 0,03 % en poids, et le plus préférablement ≥ 0,0005 % en poids à ≤ 0,02 % en poids d'au moins un agent séquestrant, de préférence le méthylglycinediacétate (MGDA) ;
- ≥ 0 % en poids à ≤ 0,5 % en poids, de préférence ≥ 0,00025 % en poids à ≤ 0,4 % en poids, plus préférablement ≥ 0,001 % en poids à ≤ 0,2 % en poids, et le plus préférablement ≥ 0,004 % en poids à ≤ 0,1 % en poids d'au moins une source alcaline, et plus préférablement d'éthanolamine ;
- un solvant de ≥ 0 % en poids à < 100 % en poids, de préférence ≥ 0,05 % en poids à ≤ 99,5 % en poids, plus préférablement ≥ 0,05 % en poids à ≤ 99 % en poids, et en outre plus préférablement ≥ 0,07 % en poids à ≤ 99 % en poids, de préférence le solvant est ajouté à 100 % en poids, et plus préférablement le solvant est de l'eau ; dans lequel
le pourcentage en poids des composants est basé sur le poids total de la composition nettoyante désinfectante et le pourcentage en poids de tous les composants de la composition est choisi de sorte qu'il ne dépasse pas 100 % en poids.
